(19) Europäisches Patentamt
European Patent Office
Office européen des brevets

(11) EP 0 910 684 B1

(12) **EUROPÄISCHE PATENTSCHRIFT**

(45) Veröffentlichungstag und Bekanntmachung des Hinweises auf die Patenterteilung:
**24.10.2001 Patentblatt 2001/43**

(21) Anmeldenummer: **97930353.4**

(22) Anmeldetag: **19.06.1997**

(51) Int Cl.[7]: **C23C 14/48**, A61K 6/06, A61C 8/00

(86) Internationale Anmeldenummer:
**PCT/DE97/01343**

(87) Internationale Veröffentlichungsnummer:
**WO 97/48835 (24.12.1997 Gazette 1997/55)**

(54) **VERFAHREN ZUR HERSTELLUNG EINES TITAN-KERAMIK-HAFTVERBUNDSYSTEMS**

PROCESS FOR PRODUCING A TITANIUM-CERAMIC ADHESIVE COMPOSITE SYSTEM

PROCEDE DE PRODUCTION D'UN SYSTEME COMPOSITE ADHESIF TITANE-CERAMIQUE

(84) Benannte Vertragsstaaten:
**AT BE CH DE DK ES FI FR GB GR IT LI NL PT SE**
Benannte Erstreckungsstaaten:
**LT LV SI**

(30) Priorität: **20.06.1996 DE 19626440**

(43) Veröffentlichungstag der Anmeldung:
**28.04.1999 Patentblatt 1999/17**

(73) Patentinhaber: **BCB Gesellschaft für biocompatible Beschichtung mbH**
**17033 Neubrandenburg (DE)**

(72) Erfinder:
• **Moormann, Andreas**
**10559 Berlin (DE)**
• **Wehnert, Lothar**
**14055 Berlin (DE)**

(74) Vertreter: **Kietzmann, Manfred et al**
**Kietzmann, Vosseberg, Röhnicke,**
**Patentanwälte-Rechtsanwalt-Partnerschaft,**
**Friedrichstrasse 95**
**IHZ P.O. Box 4**
**D-10117 Berlin (DE)**

(56) Entgegenhaltungen:
**DE-U- 9 005 995**

• **PATENT ABSTRACTS OF JAPAN vol. 014, no. 280 (C-0729), 18.Juni 1990 & JP 02 085374 A (TOSHIBA CORP), 26.März 1990,**
• **PATENT ABSTRACTS OF JAPAN vol. 012, no. 022 (C-470), 22.Januar 1988 & JP 62 174377 A (MITSUBISHI HEAVY IND LTD), 31.Juli 1987,**
• **PATENT ABSTRACTS OF JAPAN vol. 016, no. 351 (C-0968), 29.Juli 1992 & JP 04 107259 A (SHINYA IWAMOTO;OTHERS: 01), 8.April 1992,**

EP 0 910 684 B1

**Beschreibung**

Die Erfindung bezieht sich auf die Herstellung eines Titan-Keramik-Haftverbundsystems und ein daraus hergestelltes Titan-Keramik-Haftverbundsystem.

Es ist bekannt, Titan oder Titanlegierungen als Werkstoff für technische Gegenstände, die eine hohe Festigkeit sowie ein geringes Gewicht haben sollen, einzusetzen. Es wird deshalb in der Automobilindustrie sowie der Luft- und Raumfahrt zum Beispiel für Motoren und Triebwerke eingesetzt. Titan überzieht sich vor allem bei erhöhter Temperatur an der Luft mit einer fest haftenden, harten und spröden Oxidschicht. Die Oxidschicht erschwert durch Sauerstoffdiffusion das Aufbringen von anderen Stoffen auf die Oberfläche von Titan, insbesondere bei der Herstellung eines Titan-Keramik-Haftverbundsystems. Aufgrund der niedrigen Wärmeausdehnungskoeffizienten-Werte des Titans treten bei der Verblendung von Titankörpern mit Keramikmassen nicht nur Craquellé-Risse, sondern auch großflächige Abplatzungen der keramischen Schicht auf.

Nachstehend werden anhand der Entwicklung auf dem Gebiet des Einsatzes von Titan-Keramik-Haftverbundsystemen in der Dentaltechnik die Probleme näher erläutert.

Es ist bekannt, kostenaufwendige edelmetallhaltige Legierungen der zahnärztlichen Prothetik durch goldreduzierte Legierungen, Palladium-Basis-Legierungen und edelmetallfreie Legierungen zu ersetzen.

Das zunehmende Bewußtsein der Patienten hinsichtlich der allergenisierenden Wirkungen der von Zahnärzten verarbeiteten Werkstoffe sowie sich häufende allergische Reaktionen der Patienten nach Inkorporation von Zahnersatz lassen die Notwendigkeit erkennen, nach einem geeigneten Werkstoff zu suchen. Unlegiertes Titan ist ein unter diesen Gesichtspunkten geeigneter Werkstoff. Es ist als Werkstoff in der allgemeinmedizinischen und in den letzten Jahrzehnten auch in der zahnmedizinischen Implantologie und Chirurgie bewährt. Zu seinen wichtigsten Eigenschaften zählt die hohe Biokompatibilität sowie der niedrige Preis und die in der Häufigkeit seines Vorkommens begründete hohe Verfügbarkeit.

Aufgrund verschiedener werkstoffkundlicher Eigenschaften des Titans ergaben sich zunächst Schwierigkeiten bei der zahntechnischen Verarbeitung, die aber durch geeignete Maßnahmen überwunden wurden. Durch die Entwicklung von speziellen Gußsystemen sowie geeigneter Einbettmassen wurde der dentale Titanguß möglich. Die keramische Verblendbarkeit als wichtige Voraussetzung der universellen Einsetzbarkeit eines dentalen Werkstoffes gelang erst nach der Entwicklung niederschmelzender und im Wärmeausdehnungsverhalten angepaßter Keramikmassen. Klinische Nachuntersuchungen bestätigen in-vitro-Untersuchungen, bei denen Haftfestigkeitsverluste nach zyklischen Temperaturlastwechseln festgestellt wurden. Daß die Ausgangswerte der Haftfestigkeit von Titan-Keramik-Kombinationen in den verschiedenen bruchmechanischen Tests niedriger gemessen werden als bei herkömmlichen metallkeramischen Systemen, kommt erschwerend hinzu.

Als Ursache der in den klinischen Tests ermittelten Raten an schadhaften keramischen Titanverblendungen werden die invitro-gemessenen Haftfestigkeitsverluste infolge von Temperaturlastwechseln angenommen. (MOORMANN, A.: Vergleichende Untersuchungen zur Verbundfestigkeit von neun Titan-Keramik-Verbundkombinationen in Abhängigkeit von den Lagerungsbedingungen, Med Diss, Berlin 1993)

Nachdem ein einsatzfähiger Titanguß entwickelt wurde, vergrößerte sich der Anwendungsbereich des Titans in der Zahnheilkunde. Er wird außer in der Prothetik und der Implantologie auch in der Endodontie als Wurzelstiftmaterial und zu Zwecken der transdentalen Fixation eingesetzt. Darüber hinaus werden Titanlegierungen im Rahmen kieferorthopädischer Therapien mit festsitzenden Apparaturen und in der konservierenden Zahnheilkunde als Material für Inlays, Onlays und immer häufiger für Teilkronen eingesetzt.

Um ästhetischen Anforderungen zu genügen, ist die Möglichkeit der zahnfarbenen Verblendung eines zahnärztlich-prothetischen Materials wichtig. Für den universellen Einsatz eines prothetischen Werkstoffes ist die sichere keramische Verblendbarkeit eine unabdingbare Voraussetzung.

Die Hauptbestandteile dentaler keramischer Massen sind:

- ca. 80 % Kalifeldspat ($6SiO_2$-$Al_2O_3$-$K_2O$),
- ca. 15 bis 20 % Quarz ($SiO_2$),
- ca. 0 bis 10 & Tonmineralsubstanz, z. B. Kaolin.

Kalifeldspat fungiert als Flußmittel und hat Einfluß auf die Transparenz der Keramik. Kaolin steigert ebenso wie Quarz die Festigkeit der Keramik, wobei letzterer zusätzlich transparenzsteigernd wirkt.

Beim Brennvorgang durchläuft die Dentalkeramik einen Schmelzprozeß, bei dem die zugesetzten Oxide $SiO_2$ und $B_2O_3$ eine glasartige Matrix bilden, in die Leuzitkristalle eingelagert sind. Nicht voll aufgeschmolzene Bestandteile liegen als Sinterphase vor. Dementsprechend besteht gebrannte dentale Metallkeramik aus Glas-, Sinter- und Kristallphase.

Der Notwendigkeit der Abstimmung der Wärmeausdehnungskoeffizienten des Metalls und der Keramik folgend, ist eine Modifikation der Kristallphase erforderlich. Der Umstand, daß Metalle im Vergleich zu Glas einen sehr hohen Wärmeausdehnungskoeffizienten besitzen, bedingt, daß in der keramischen Masse das Verhältnis von Glas zu Keramik (Leuzit) auf die jeweilige Legierung abgestimmt ist.

Im Zusammenhang mit dem Einsatz von Reintitan für die Herstellung von Zahnersatz wurden neuartige, speziell auf die Anforderungen des Titans abgestimmte Keramikmassen entwickelt.

Wegen der im Vergleich mit herkömmlichen dentalen Aufbrennlegierungen niedrigen Werte der Wärmeausdehnungskoeffizienten, der hohen Sauerstoffaffinität und der allotropen Umwandlung der Gitterstruktur bei 882,5 °C mußten Keramikmassen mit im Vergleich zu den üblichen Keramikmassen veränderten Eigenschaften entwickelt werden. Aufgrund der niedrigen Wärmeausdehnungskoeffizienten-Werte des Titans würden bei der Verblendung von Titangerüsten mit herkömmlichen keramischen Verblendmaterialien nicht nur Craquellé-Risse, sondern auch großflächige Abplatzungen der keramischen Schicht auftreten. (LINDIGKEIT, J.: Werkstoffkunde und Technologie, In: SIEBERT, G. K.: Dentallegierungen in der zahnärztlichen Praxis, Hanser, München - Wien 1989)

Eine Anpassung des Wärmeausdehnungskoeffizienten mit einer Herabsetzung des Wertes um 30 % war über eine Vergrößerung des Glasanteils bei Ersatz des Leuzits durch das Aluminiumsilikat Mullit möglich.

Eine um 150 bis 200 °C niedrigere Sintertemperatur wird über eine Erhöhung des Natriumoxidgehaltes ($Na_2O$) bei Herabsetzung des Aluminiumoxidgehaltes ($Al_2O_3$) erreicht.

Die hohe Sauerstoffaffinität bzw. Oxidationsneigung erfordert den Einsatz von speziellen Bondern, die bereits auf der Titanoberfläche vorhandene Oxide auflösen bzw. umschließen sollen und durch ihre glasartige Beschaffenheit eine Versiegelung gegen weitere Oxidation bewirken.

Die beschriebenen Änderungen hinsichtlich der Zusammensetzung der titankeramischen Verblendmaterialien wirken sich weder auf ihre Hydrolysebeständigkeit noch auf ihre Biegefestigkeit aus.

Analog der relativen Unkenntnis der genauen Mechanismen der Haftvermittlung zwischen herkömmlichen Aufbrennlegierungen und den keramischen Massen existieren nur unvollständige Vorstellungen über die Bindung zwischen Titan und den entsprechenden keramischen Verblendmaterialien. Dementsprechend gegensätzlich wird dieses Thema in der einschlägigen Literatur diskutiert.

Neben der allgemein gehaltenen Annahme, daß die im vorangegangenen Abschnitt beschriebenen Mechanismen auch an der Bindung Titan - Keramik beteiligt sind, gehen die Untersuchungen auf unterschiedliche und zum Teil sehr spezielle Aspekte der entsprechenden Mechanismen ein.

MOORMANN geht in seiner Med. Diss., Berlin 1993 davon aus, daß die Ausbildung einer oxidischen, schuppig-kristallinen Zwischenschicht im Bereich der Titan-Keramik-Kontaktzone (vermutlich im wesentlichen aus $Ti_5Si_3$ und vor allem im Bereich der oberflächlichen Titanschicht aus Oxiden bestehend) zunächst am Zustandekommen des Titan-Keramik-Haftverbundes beteiligt ist. Gleichwohl erfährt dieser Bereich aufgrund der hohen Reaktivität des Titans auch nach Abschluß des keramischen Brennvorgangs chemische Veränderungen im Sinne einer fortlaufenden

Sauerstoffversprödung der oberflächlichen Titanschicht, worin MOORMANN auf der anderen Seite die Ursache für das Versagen des Verbundes vermutet.

Neben der Entwicklung von geeigneten Titankeramiken mit, den Erfordernissen angepaßten, niedrigeren Sintertemperaturen und geringeren thermischen Ausdehnungskoeffizienten wurden, wie dargelegt, spezielle Bonder entwickelt, die durch ihre reduzierenden Eigenschaften vorhandene Oxidschichten auf der Titanoberfläche auflösen, respektive die Oxide umschließen und durch eine Art Versiegelung die Bildung einer neuen Oxidschicht in der Metall-Keramik-Grenzschicht während des Aufbrennvorganges verhindern sollen.

Ebenfalls unter der Zielsetzung, die Oxidbildung des Titans während des keramischen Brandes zu vermeiden, wurden keramische Verblendungen von Titan unter Schutzgasatmosphäre durchgeführt.

Zur Vermeidung verbundschwächender Einflüsse der α-case wird empfohlen, eine für den dentalen Guß speziell entwickelte Einbettmasse zur Vermeidung der α-case Schichtstärke zu benutzen und diese Schicht auf der zu verblendenden Fläche vollständig zu entfernen.

Zur vollständigen Vermeidung einer α-case und Erlangung eines lunkerfreien Werkstückes ist es möglich, unter Anwendung von CAD/CAM-Techniken, Funkenerosion bzw. einer Kombination beider Techniken zahntechnische Werkstücke aus vorgefertigten Titan-Halbzeugkörpern herauszuarbeiten.

Inwiefern Elemente, welche die α-Phase des Titans stabilisieren, den Titan-Keramik-Haftverbund beeinflussen, untersuchte MOORMANN anhand einer Titan-Aluminium-Legierung. (MOORMANN,A.: Vergleichende Untersuchungen zur Verbundfestigkeit von neun Titan-Keramik-Verbundkombinationen in Abhängigkeit von den Lagerunsgbedingungen Med Diss, Berlin 1993)

POTTHOFF untersuchte die Anwendung des PROBOND-Verfahrens auf Brückengerüsten aus Titan. (POTTHOFF, D.: Biegefestigkeits- und Randspaltuntersuchung von metallkeramischen Seitenzahnbrücken-Probondverfahren und konventionelles Verfahren, Zahnmed Diss, FU-Berlin 1994)

Die Möglichkeit, mit Hilfe von mechanischer Oberflächenbearbeitung durch Feinschliff mit verschiedenen Körnungen (F 80 und F 220) und durch anschließendes Strahlen mit Korund (Korngröße 250 μm) den Titan-Keramik-Haftverbund zu verbessern, wurde von TESCH et al untersucht. (TESCH, U.; PÄSSLER, V.; MANN, E.: Untersuchungen zum Titan-Keramik Verbund dentallabor XLI, 1/93, 71-74) ECKMANN untersuchte den Einfluß mechanischer Retentionen (Retentionsperlen) bzw. einer oberflächlichen Titan-Plasma-beschichtung auf den Haftverbund zwischen Titan und Keramik. (ECKMANN, St.: Untersuchungen zur Biegefestigkeit des Titan-Keramik-Verbundes bei Brücken in Abhängigkeit von der Oberflächenbearbeitung sowie zur Paßgenauigkeit Zahnmed Diss, Berlin 1994)

DERAND und HERO unternahmen den Versuch, den Haftverbund durch die Anwendung eines speziellen Goldbonders zu verbessern. DERAND, T.; HERO, H.: Bond strength of porcelain on cast versus wrought titanium Scand J Dent Res 100, 184-188 (1992)

KRUSE und BAUMANN untersuchten den Einfluß von variierenden Brenntemperaturen auf die Haftfestigkeit von Keramik auf Titan. (KRUSE, N.: Untersuchung zur Abscherfestigkeit des Titan-Keramik-Verbundes bei fünf Titankeramischen Systemen in Abhängigkeit verschiedener Aufbrenntemperaturen -Eine in -Vitro-Studie-, Zahnmed Diss, Berlin 1995; BAUMANN, W.: Bruchmechanische Haftfestigkeitsbestimmung von Verblendmetall-Keramik auf Titan, Med Diss, Aachen 1992)

Trotz dieser großen Anzahl an Versuchen, den Titan-Keramik-Haftverbund zu verbessern, ist es nicht gelungen, eine den zahnmedizinischen Aufbrennlegierungen gleichwertige Sicherheit zu erzielen, was durch verschiedene klinische Longitudinalstudien bestätigt wird.

Der Erfindung liegt die Aufgabe zugrunde, ein Verfahren zur Herstellung eines Titan-Keramik-Haftverbundsystems und ein daraus hergestelltes Titan-Keramik-Haftverbundsystem zu schaffen, welches geeignet ist, die Haftfestigkeit von Reintitan oder Titanlegierungen für bestimmte Keramiken auf einem Körper aus Reintitan oder einer Titanlegierung zu verbessern.

Erfindungsgemäß wird die Aufgabe dadurch verbessert, daß in die Oberfläche eines Körpers aus Reintitan oder einer Titanlegierung durch eine Ionenimplantation mit Ionenstrahlen zwischen die Atome des Titans oder die Atome der Titanlegierung Siliziumionen eingebracht werden, durch die in der Oberfläche des Körpers in der Durchdringungsschicht der Ionenimplantation eine Titan-Silizium-Schicht ausgebildet wird, auf die kristalline, nichtmetallisch-anorganische Werkstoffe thermisch aufgetragen werden und mit den Werkstoffen ein Haftverbund hergestellt wird.

Vorzugsweise werden die Siliziumionen in Form von Siliziumaggregaten in die Titan-Silizium-Schicht eingelagert.

Zweckmäßigerweise bestehen die kristallinen, nichtmetallisch-anorganischen Werkstoffe aus glaskeramischen Werkstoffen, nichtoxidischen keramischen Werkstoffen oder oxidischen keramischen Werkstoffen.

Als eine Titanlegierung kann eine Titan-Vanadium-Aluminium-Legierung folgender Zusammensetzung eingesetzt werden:

Ti-6Al-4V

Es ist möglich, als Titanlegierung eine den speziellen Anforderungen des Einsatzgebietes und der möglichen Verfahrenstechnik entsprechende Titanlegierung zu verwenden.

Vorteilhaft ist es, wenn die Innenimplantation von Siliziumionen in die Oberfläche des Körpers aus Reintitan oder einer Titanlegierung mit einer Ionendosis von $1 \times 10^8$ bis $1 \times 10^{18}$ Atomen/cm$^2$ und einer Ionenenergie von 30 bis 400 KeV durchgeführt wird.

In bevorzugter Weise wird die Ionenimplantation von Siliziumionen in die Oberfläche des Körpers aus Reintitan oder einer Titanlegierung mit einer Ionendosis von $9 \times 10^{16}$ Atomen/cm$^2$ und einer Ionenenergie von 150 KeV durchgeführt.

Durch die erfindungsgemäße Lösung wird erreicht, daß die Haftfestigkeit des für Reintitan oder eine Titanlegierung bestimmten kristallinen, nichtmetallisch-anorganischen Werkstoffes erhöht wird.

Dabei werden insbesondere die Temperaturlastwechsel, wie sie sich bei längerem Gebrauch von daraus gefertigten Werkstücken ergeben, verringert.

Eine bevorzugte Ausbildung der Erfindung besteht darin, daß zur Verwendung für einen Zahnersatz in die Oberfläche eines Körpers aus Reintitan durch eine Ionenimplantation mit Ionenstrahlen zwischen die Atome des Titans Siliziumionen eingebracht werden, durch die in der Oberfläche des Körpers in der Durchdringungsschicht der Ionenimplantation eine Titan-Silizium-Schicht ausgebildet wird, auf die eine für die Verblendung von Titan bestimmte Dentalkeramik aufgebrannt wird.

Für die Ionenimplantation von Siliziumionen in die Oberfläche des Körpers aus Reintitan ist eine Ionendosis von $1 \times 10^{12}$ bis $1 \times 10^{18}$ Atomen/cm$^2$ und eine Ionenenergie von 30 bis 400 KeV besonders geeignet.

Vorzugsweise wird die Ionenimplantation von Siliziumionen in die Oberfläche des Körpers aus Reintitan mit einer Ionendosis von $3 \times 10^{17}$ Atomen/cm$^2$ und einer Ionenenergie von 150 KeV durchgeführt.

Entsprechend den durchgeführten Versuchen kann das Reintitan folgende Anteile (Angaben in Masse-%) enthalten:

| | |
|---|---|
| $O_{max}$ | 0,12 |
| $N_{max}$ | 0,05 |
| $C_{max}$ | 0,06 |

(fortgesetzt)

| $H_{max}$ | 0,013 |
|-----------|-------|
| Ti | Rest |

Als besonders vorteilhaft hat sich erwiesen, daß vor der Ionenimplantation der Oberfläche des Körpers aus Reintitan die oberflächige Titanoxidschicht durch eine spanabhebende Bearbeitung entfernt wird und anschließend durch gemahlenes, monokristallines Silizium ($Si_{mon}$) mit einer Maschgröße von 50 bis 300 µm unter einer Schutzgasatmosphäre aufgerauht wird.

Es ist auch möglich, daß nach der Entfernung der Titanoxidschicht von der Oberfläche des Körpers aus Reintitan die Oberfläche durch Abstrahlen mit Korund ($\alpha$-$Al_2O_3$) der Korngröße 50 - 250 µm aufgerauht wird.

Durch die erfindungsgemäße Lösung ist es möglich, daß der Körper aus Reintitan vor der Ionenimplantation vollständig als ein Grundkörper für einen Zahnersatz gestaltet wird und außer dem Aufbrennen der Dentalkeramik keine weitere Bearbeitung des Körpers nach der Ausbildung der Titan-Silizium-Schicht in der Oberfläche des Körpers durchgeführt wird.

Eine bevorzugte Ausführungsform besteht darin, daß in der gesamten Oberfläche des als Grundkörper für einen Zahnersatz gestalteten Körpers aus Reintitan die Titan-Silizium-Schicht ausgebildet wird und die für die Verblendung von Titan bestimmte Dentalkeramik auf einzelne Abschnitte der Oberfläche aufgebrannt wird, wobei die Dentalkeramik mindestens auf die, die Zahnbereiche und die Bereiche des Schleimhautkontaktes bildenden, Abschnitte des Grundkörpers für einen Zahnersatz aufgebrannt wird.

Auf diese Weise wird der Aufwand bei der Herstellung des Titan-Keramik-Haftverbundes für Zahnersatz gering gehalten. Gleichzeitig wird ein Austreten von Titanionen aus dem Körper aus Reintitan in den Mundbereich geblockt.

Die für die Verblendung von Titan bestimmte Dentalkeramik wird zweckmäßigerweise in vier Brennzyklen

1. Zyklus: Bonder und/oder Washbrand-Masse
2. Zyklus: Grundmassebrand
3. Zyklus: Dentinbrand
4. Zyklus: Glanzbrand

auf die Titan-Silizium-Schicht aufgebrannt.

Auf diese Weise ist es möglich, auf die in der Oberfläche gebildete Titan-Silizium-Schicht in üblicher Weise nach den Hinweisen des Herstellers die Dentalkeramik aufzubauen. Eine Veränderung der Brennweise ist nicht erforderlich.

Eine weitere bevorzugte Ausbildung der Erfindung besteht darin, daß zur Verwendung in einem Hochtemperaturbereich von 600 bis 3600 °C in die Oberfläche eines Körpers aus Reintitan oder einer Titanlegierung durch eine Ionenimplantation mit Ionenstrahlen zwischen die Atome des Titans oder die Atome der Titanlegierung Siliziumionen eingebracht werden, durch die in der Oberfläche des Körpers in der Durchdringungsschicht der Ionenimplantation eine Titan-Silizium-Schicht ausgebildet wird, auf die kristalline, nichtmetallisch-anorganische Werkstoffe thermisch aufgetragen werden und mit den Werkstoffen ein Haftverbundsystem hergestellt wird.

Zweckmäßigerweise werden die Siliziumionen in Form von Siliziumaggregaten in die Titan-Siliziumschicht eingelagert.

Die kristallinen, nichtmetallisch-anorganischen Werkstoffe können aus glaskeramischen Werkstoffen, nichtoxidischen keramischen Werkstoffen oder oxidischen keramischen Werkstoffen bestehen.

Vorteilhaft ist es, wenn die Ionenimplantation von Siliziumionen in die Oberfläche des Körpers aus Reintitan oder einer Titanlegierung mit einer Ionendosis von $1 \times 10^8$ bis $1 \times 10^{18}$ Atomen/cm$^2$ und einer Ionenenergie von 30 bis 400 KeV durchgeführt wird.

Hierbei kann die Ionenimplantation von Siliziumionen in die Oberfläche des Körpers aus Reintitan oder einer Titanlegierung mit einer Ionendosis von $9 \times 10^{16}$ Atomen/cm$^2$ und einer Ionenenergie von 150 KeV durchgeführt werden.

Vorzugsweise wird der Körper aus Reintitan oder einer Titanlegierung vor der Ionenimplantation als ein Werkstück zur Verwendung in einem Hochtemperaturbereich von 600°C bis 3600°C ausgebildet und außer dem thermischen Auftragen eines kristallinen, nichtmetallisch-anorganischen Werkstoffes keine weitere Bearbeitung des Körpers nach der Ausbildung der Titan-Silizium-Schicht in der Oberfläche des Körpers durchgeführt.

Das Werkstück kann in Motoren und Triebwerken des Kraftfahrzeugbaus sowie der Luft- und Raumfahrt eingesetzt werden.

In weiterer Ausbildung umfaßt die Erfindung ein Titan-Keramik-Haftverbundsystem, wobei die Oberfläche eines Körpers aus Reintitan oder einer Titanlegierung als eine Titan-Silizium-Schicht ausgebildet ist, wobei die Siliziumionen durch eine Ionenimplantation zwischen die Atome des Titans oder die Atome der Titanlegierung eingebracht sind und wobei auf die Titan-Silizium-Schicht ein kristalliner, nichtmetallisch-anorganischer Werkstoff thermisch aufgetragen ist.

Vorzugsweise sind die Siliziumionen zur Ausbildung der Titan-Silizium-Schicht in der Oberfläche des Körpers aus Reintitan oder einer Titanlegierung mit einer Ionendosis von $1 \times 10^8$ bis $1 \times 10^{18}$ Atomen/cm$^2$ und einer Ionenenergie von 30 bis 400 KeV eingebracht.

In bevorzugter Weise können dabei die Siliziumionen in der Oberfläche des Körpers aus Reintitan oder einer Titanlegierung mit einer Ionendosis von $9 \times 10^{16}$ Atomen/cm$^2$ und einer Ionenenergie von 150 KeV eingebracht sein.

Das erfindungsgemäße Titan-Keramik-Haftverbundsystem kann zur Verwendung für einen Zahnersatz ausgebildet sein, wobei die Oberfläche eines Körpers aus Reintitan als eine Titan-Silizium-Schicht ausgebildet ist, wobei Siliziumionen durch eine Ionenimplantation zwischen die Titanatome des Reintitans eingebracht sind und wobei auf die Titan-Silizium-Schicht eine für die Verblendung von Titan bestimmte Dentalkeramik aufgebrannt ist.

Vorzugsweise sind die Siliziumionen zur Ausbildung der Titan-Silizium-Schicht in der Oberfläche des Körpers aus Reintitan mit einer Ionendosis von $1 \times 10^{12}$ bis $1 \times 10^{18}$ Atomen/cm$^2$ an Siliziumionen und einer Ionenenergie von 30 bis 400 KeV eingebracht.

Hierbei können die Siliziumionen zur Ausbildung der Titan-Silizium-Schicht in der Oberfläche des Körpers aus Reintitan mit einer Ionendosis von $3 \times 10^{17}$ Atomen/cm$^2$ und einer Ionenenergie von 150 KeV eingebracht sein.

Es ist möglich, daß das Reintitan folgende Zusammensetzung (Angaben in Masse-%) aufweist:

| | |
|---|---|
| $O_{max}$ | 0,12 |
| $N_{max}$ | 0,05 |
| $C_{max}$ | 0,06 |
| $H_{max}$ | 0,013 |
| Ti | Rest |

Vorteilhafterweise ist der Körper aus Reintitan vor der Ausbildung seiner Oberfläche als eine Titan-Silizium-Schicht vollständig als ein Grundkörper für einen Zahnersatz gestaltet, auf dem nach der Ausbildung der Titan-Silizium-Schicht als einzige Bearbeitung die Dentalkeramik durch Aufbrennen aufgebracht ist.

Eine bevorzugte Ausführung des Titan-Keramik-Haftverbundsystems besteht darin, daß in der gesamten Oberfläche des als Grundkörper für einen Zahnersatz gestalteten Körpers aus Reintitan die Titan-Silizium-Schicht ausgebildet ist und die für die Verblendung von Titan bestimmte Dentalkeramik auf einzelne Abschnitte der Oberfläche aufgebrannt ist, wobei die Dentalkeramik mindestens auf die, die Zahnbereiche und die Bereiche des Schleimhautkontaktes bildenden, Abschnitte des Grundkörpers für einen Zahnersatz aufgebrannt ist.

In weiterer Ausbildung der Erfindung ist das Titan-Keramik-Haftverbundsystem so ausgebildet, daß zur Verwendung in einem Hochtemperaturbereich von 600 bis 3600 °C die Oberfläche eines Körpers aus Reintitan oder einer Titanlegierung als eine Titan-Silizium-Schicht ausgebildet ist, wobei die Siliziumionen durch eine Ionenimplantation zwischen die Atome des Titans oder die Atome der Titanlegierung eingebracht sind und wobei auf die Titan-Silizium-Schicht ein kristalliner, nichtmetallisch-anorganischer Werkstoff thermisch aufgetragen ist.

Die Siliziumionen können in Form von Siliziumaggregaten in die Titan-Silizium-Schicht eingelagert sein.

Dabei ist es zweckmäßig, daß die kristallinen, nichtmetallisch-anorganischen Werkstoffe aus glaskeramischen Werkstoffen, nichtoxidischen keramischen Werkstoffen oder oxidischen keramischen Werkstoffen bestehen.

Die Ionenimplantation von Siliziumionen in die Oberfläche des Körpers aus Reintitan oder einer Titanlegierung kann mit einer Ionendosis von $1 \times 10^8$ bis $1 \times 10^{18}$ Atomen/cm$^2$ und einer Ionenenergie von 30 bis 400 KeV durchgeführt sein.

Zweckmäßig ist es, wenn die Ionenimplantation von Siliziumionen in die Oberfläche des Körpers aus Reintitan oder einer Titanlegierung mit einer Ionendosis von $9 \times 10^{16}$ Atomen/cm$^2$ und einer Ionenenergie von 150 KeV durchgeführt ist.

In bevorzugter Weise besteht der Körper aus Reintitan oder einer Titanlegierung, wobei vor der Ionenimplantation der Körper als ein Werkstück zur Verwendung in einem Hochtemperaturbereich von 600 bis 3600 °C ausgebildet ist und außer dem thermischen Auftragen eines kristallinen, nichtmetallisch-anorganischen Werkstoffes keine weitere Bearbeitung des Körpers nach der Ausbildung der Titan-Silizium-Schicht in der Oberfläche des Körpers erfolgt.

Dabei kann das Werkstück in Motoren und Triebwerken des Kraftfahrzeugbaus sowie der Luft- und Raumfahrt eingesetzt sein.

Die Erfindung wird an einem Ausführungsbeispiel erläutert. In der zugehörigen Zeichnung zeigen:

Fig. 1: Oberflächenmodifikation durch Ionenimplantation in schematischer Darstellung,

Fig. 2:     graphische Darstellung der Medianwerte der Verbundfestigkeiten in MPa für die Kombination von $\alpha$-Al$_2$O$_3$ gestrahltem Titan und TIBOND,

Fig. 3:     graphische Darstellung der Medianwerte der Verbundfestigkeiten in MPa für die Kombination von $\alpha$-Al$_2$O$_3$-gestrahltem Titan und VITA TITANKERAMIK,

Fig. 4:     graphische Darstellung der Medianwerte der Verbundfestigkeiten von nicht $\alpha$-Al$_2$O$_3$-gestrahltem Titan und TIBOND,

Fig. 5:     graphische Darstellung der Medianwerte der Verbundfestigkeiten in MPa für die Kombination von nicht $\alpha$-Al$_2$O$_3$-gestrahltem Titan und VITA TITANKERAMIK,

Fig. 6:     Anordnung der Meßpunkte der EDAX-Analysen,

Fig. 7:     REM-Aufnahme der Kombination Titan $\alpha$-Al$_2$O$_3$-gestrahlt/ TIBOND, TC, Vergrößerung: 3300 : 1 mit Ionenimplantation,

Fig. 8:     REM-Aufnahme der Kombination Titan $\alpha$-Al$_2$O$_3$-gestrahlt/ TIBOND, TC, Vergrößerung: 3200 : 1 ohne Ionenimplantation,

Fig. 9:     REM-Aufnahme der Kombination Titan $\alpha$-Al$_2$O$_3$-gestrahlt/ VITA TITANKERAMIK, TC, Vergrößerung: 3200 : 1 mit Ionenimplantation,

Fig. 10:    REM-Aufnahme der Kombination Titan $\alpha$-Al$_2$O$_3$-gestrahlt/ VITA TITANKERAMIK, TC, Vergrößerung: 3200 : 1 ohne Ionenimplantation,

Fig. 11:    REM-Aufnahme der Kombination Titan nicht $\alpha$-Al$_2$O$_3$-gestrahlt/TIBOND, TC, Vergrößerung: 2000 : 1 mit Ionenimplantation,

Fig.12:     REM-Aufnahme der Kombination Titan nicht $\alpha$-Al$_2$O$_3$-gestrahlt/TIBOND, TC, Vergrößerung: 2000 : 1 ohne Ionenimplantation,

Fig.13:     REM-Aufnahme der Kombination Titan nicht -$\alpha$-Al$_2$O$_3$-gestrahlt/VITA TITANKERAMIK, TC, Vergrößerung: 2000 : 1 mit Ionenimplantation,

Fig.14:     REM-Aufnahme der Kombination Titan nicht $\alpha$-Al$_2$O$_3$-gestrahlt/VITA TITANKERAMIK, TC, Vergrößerung: 2000 : 1 ohne Ionenimplantation.

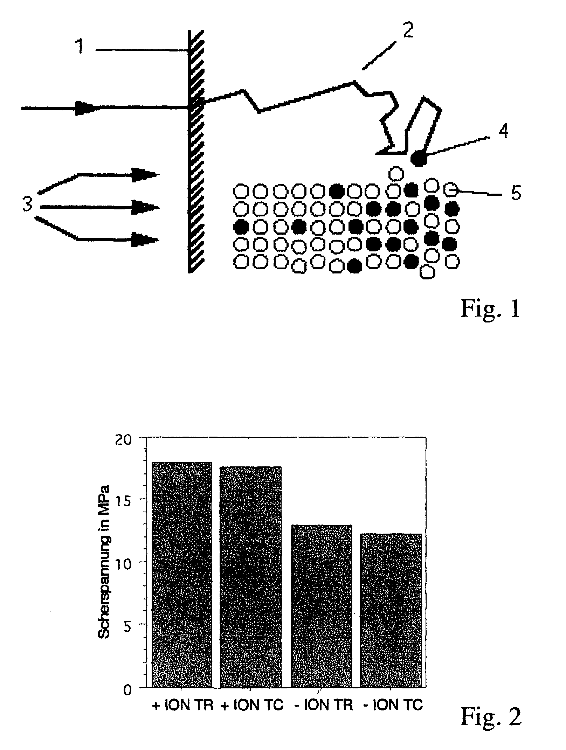

In Fig. 1 ist die Behandlung von Titan durch eine Ionenimplantation gezeigt. Bei der Ionenimplantation trifft ein energiereiches Ion auf einen Festkörper und es kommt zu verschiedenen Wechselwirkungen mit seinen oberflächennah angesiedelten Atomen. Unter Titan wird in den nachstehenden Ausführungen sowohl Reintitan als auch eine Titanlegierung verstanden.

Durch Stöße zwischen den einfallenden Ionen und den Elektronen und Atomkernen des beschossenen Materials werden die Ionen aus ihrer Bahnrichtung abgelenkt, beschreiben dabei einen Polygonzug als Bahn und kommen infolge des Verlustes ihrer kinetischen Energie statistisch verteilt zur Ruhe.

Bei dem Verfahren zur Herstellung eines Titan-Keramik-Haftverbundsystems werden bei der Ionenimplantation in eine Oberfläche 1 eines Körpers aus Titan durch einen Ionenstrahl 3 Siliziumionen 4 zwischen die Titanatome 5 in einer Durchdringungszone eingebracht. In der Fig. 1 sind die Siliziumionen 4 schwarz und die Titanatome 5 weiß dargestellt. Es entsteht eine Titan-Silizium-Schicht 2. Die Ausbildung der Titan-Silizium-Schicht 2 ist allerdings von der Masse der Siliziumionen 4 und der Titanatome 5 sowie der Implantationsparameter, nämlich Ionenenergien und Ionendosis, abhängig.

Die Ionenimplantation von Siliziumionen 4 in die Oberfläche 1 des Körpers aus Titan erfolgt mit einer Ionendosis von 1 x 10$^8$ bis 1 x 10$^{18}$ Atomen/cm$^2$ und einer Ionenenergie von 30 bis 400 KeV. Vorteilhaft ist dabei, mit einer Ionendosis von 9 x 10$^{18}$ Atomen/cm$^2$ und einer Ionenenergie von 150 KeV zu arbeiten.

Die durch die Ionenimplantation in der Oberfläche 1 des Titans als Fremdelementenschicht eingebrachte Titan-Silizium-Schicht 2 bildet keine zusätzliche Schicht auf der Oberfläche 1 des Titans, wodurch die Form und die Paßgenauigkeit des aus dem Titan-Keramik-Haftverbundsystems hergestellten Werkstückes unverändert bleibt.

Weiterhin sind Veränderungen der physikalisch-chemischen Eigenschaften des gesamten Werkstückes durch die Implantation der Siliziumionen 4 ausgeschlossen. Vielmehr ist ausschließlich die Titan-Keramik-Kontaktzone den gewünschten Modifikationen ausgesetzt. Bei der Ionenimplantation handelt es sich chemisch um einen "Nichtgleichgewichtsprozeß", das heißt das Einbringen des Fremdelementes unterliegt keinen thermodynamischen Begrenzungen durch Löslichkeitsgleichgewichte oder atomare Diffusionsraten. Dementsprechend sind in diesem Verfahren auf konventionelle Weise unlösliche Elemente miteinander vermischbar und mit Bezug auf das ausgewählte Siliziumion 4 deutlich höhere Konzentrationen als seine Löslichkeit von weniger als 1 Atom-% in Titan entsprechend erzielbar.

Mit der Ionenimplantation ist eine für die Passivierung der Oberfläche 1 des Titans ausreichende Siliziumkonzentration erreichbar. Hierbei ist es vorteilhaft, die Siliziumionen 4 in Form von Siliziumaggregaten in die Titan-Silizium-Schicht 2 einzulagern.

Die Temperatur des Festkörpers aus Titan während der Ionenimplantation ist kontrollierbar und kann auf Temperaturen unter 100 °C gehalten werden, wodurch thermische Einflüsse, die durch die mögliche allotrope Umwandlung der Titanelementarkörper von $\alpha$- in $\beta$-Kristalle die für den Einsatz von Werkstücken mit hoher Paßgenauigkeit diese verändern könnten, ausgeschaltet sind.

Die durch die Ionenimplantation gebildete Titan-Silizium-Schicht 2 weist eine hervorragende Haftfestigkeit auf. Dabei wird durch die weitgehende Unabhängigkeit von thermodynamischen Gesetzmäßigkeiten ein "Einfrieren" der entstandenen chemischen Verbindung ermöglicht.

Auf die so vorbereitete Oberfläche 1 des Titans werden kristalline, nichtmetallisch-anorganische Werkstoffe thermisch aufgetragen und mit den Werkstoffen ein Haftverbund hergestellt.

Geeignete kristalline, nichtmetallisch-anorganische Werkstoffe sind glaskeramische Werkstoffe, nichtoxidisch keramische Werkstoffe oder oxidisch keramische Werkstoffe. Diese Werkstoffe sind auf die durch Ionenimplantation von Siliziumionen 4 behandelte Oberfläche 1 des Körpers aus Titan, d. h. Reintitan oder eine Titanlegierung, auftragbar und weisen gegenüber dem Auftrag auf unbehandelte Körper eine wesentlich höhere Haftfestigkeit auf. Dieses wird nachstehend noch an dem Beispiel des Einsatzes des erfindungsgemäßen Titan-Keramik-Haftverbundsystems näher erläutert.

Eine geeignete Titanlegierung ist eine Titan-Vanadium-Aluminium-Legierung folgender Zusammensetzung: Ti-6Al-4V

Hierbei ist davon auszugehen, daß als Titanlegierung eine den speziellen Anforderungen des Einsatzgebietes und der möglichen Verfahrenstechnik entsprechende Titanlegierung verwendet wird. Das Titan-Keramik-Haftverbundsystem bildet die Oberfläche 1 von Körpern, die bereits die Form des zum Einsatz kommenden fertigen Werkstückes aufweisen. Dieses ist dadurch möglich, da, wie vorstehend beschrieben, keine zusätzlichen Schichten auf die Oberfläche 1 des Titans aufgebracht werden und die Temperatur des Grundkörpers aus Titan während der Ionenimplantation auf unter 100 °C gehalten wird, so daß keine thermisch bedingten Verformungen auftreten können.

Versuche haben ergeben, daß das Titan-Keramik-Haftverbundsystem besonders zur Verwendung für einen Zahnersatz geeignet ist.

Bei dem Verfahren zur Herstellung eines Titan-Keramik-Haftverbundsystems zur Verwendung für einen Zahnersatz werden in der Oberfläche 1 eines Körpers aus Reintitan durch eine Ionenimplantation mit Ionenstrahlen 3 zwischen die Atome 5 des Titans Siliziumionen 4 eingebracht, durch die in der Oberfläche 1 des Körpers in der Durchdringungsschicht der Ionenimplantation eine Titan-Silizium-Schicht 2 ausgebildet wird.

Der Einsatz von Reintitan ist aus zahnmedizinischen Gründen erforderlich.

Auf die so vorbereitete Oberfläche 1 des Titans werden eine Dentalkeramikmasse, die speziell für die Verblendung von Titan bestimmt ist, nach Herstellerangaben aufgebrannt.

Dabei sind für die Herstellung des Titan-Keramik-Haftverbundsystems für einen Zahnersatz grundsätzlich vier Brennzyklen erforderlich. Die Brennzyklen sind:

1. Zyklus: Bonder und/oder Washbrand-Masse
2. Zyklus: Grundmassebrand
3. Zyklus: Dentinbrand
4. Zyklus: Glanzbrand

Es sind auch noch weitere Brennzyklen, wie beispielsweise ein Malfarbenbrand, möglich.

Nachstehend wird an einzelnen Beispielen die Versuchsdurchführung mit deren Ergebnissen bei der Herstellung des Titan-Keramik-Haftverbundsystems für Zahnersatz erläutert.

Beispiel 1: Vorbereitung des Titans

In den vorliegenden Untersuchungen wurde unlegiertes Titan Ti 2 nach Tabelle 1 verwendet.

Tabelle 1:

| Chemische Zusammensetzung von Reintitan nach DIN 17850 | | | | | | |
|---|---|---|---|---|---|---|
| Werkstoff | | chemische Zusammensetzung (Masse-%) | | | | |
| Kurzzeichen | Nummer | $O_{max}$ | $N_{max}$ | $C_{max}$ | $H_{max}$ | Ti |
| Ti 1 | 3,07025 | 0,12 | 0,05 | 0,06 | 0,013 | Rest |
| Ti 2 | 3,07035 | 0,18 | 0,05 | 0,06 | 0,013 | Rest |
| Ti 3 | 3,07055 | 0,25 | 0,05 | 0,06 | 0,013 | Rest |
| Ti 4 | 3,07065 | 0,35 | 0,05 | 0,06 | 0,013 | Rest |

Die physikalischen Eigenschaften von unlegiertem Titan sind in der Tabelle 2 enthalten.

Tabelle 2:

| Physikalische Eigenschaften von unlegiertem Titan | |
|---|---|
| Ordnungszahl | 22 |
| Atomgewicht | 47,8 |
| Dichte (g/ccm) | 4,51 |
| Schmelzpunkt (°C) | 1688 |
| Siedepunkt (°C) | 3260 |
| Härte (n.Vickers) | 80-105 |
| Zugfestigkeit (MPa) kaltverformt Guß | 450 850 |
| Bruchdehnung (%) | 15-20 |
| Wärmeausdehnungskoeffizient (1/K) | $9,6 \times 10^{-6}$ |
| Wärmeleitfähigkeit (W/mK) | 21,4 |

Für den Schertest nach SCHMITZ-SCHULMEYER erfolgte der Einsatz von unlegiertem und gezogenem Titan Ti 2 in Form von länglichen Quadern mit einer Kantenlänge von 5,9 x 5,9 x 1000 mm.

Von diesen Quadern wurden quadratische Würfel mit der Kantenlänge 5,9 x 5,9 mm abgeschnitten. Der für die vorliegende Untersuchung entscheidende Vorteil ist die Erlangung eines lunkerfreien Werkstückes mit einer α-case freien Oberfläche. Die Schwierigkeit der definierten Entfernung dieser Schicht und daraus möglicherweise folgende Inkongruenzen bezüglich der Maßhaltigkeit der Probekörper werden durch diese Vorgehensweise vermieden.

Die zur keramischen Verblendung vorgesehenen Flächen wurden zwei unterschiedlichen mechanischen Oberflächenbearbeitungen unterzogen.

Zunächst wurde die Oberfläche mit einem kreuzverzahnten Fräser unter einem Materialabtrag in definierter Stärke bearbeitet, um die oberflächige Titanoxidschicht zu entfernen. Anschließend erfolgte bei einer Hälfte der Probekörper ein Aufrauhen durch Abstrahlen mit Korund der Korngröße 250 µm. Damit liegt die Korngröße an der oberen Grenze des Bereiches von 50 bis 250 µm.

Weitere Versuche haben ergeben, daß nach dem Entfernen der oberflächigen Titanoxidschicht das Aufrauhen unter Einsatz von gemahlenem, monokristallinem Silizium $Si_{mon}$ mit einer Maschgröße von 50 bis 300 µm unter einer Schutzgasatmosphäre erfolgen kann.

Bei der anderen Hälfte der Probekörper unterblieb ein derartiges Aufrauhen der Oberfläche 1 mit der damit verbundenen Vergrößerung der Titan-Keramik-Haftfläche und Erhöhung der mechanischen Haftung. Mit Hilfe dieser Vergleichscharge sollte festgestellt werden, ob sich die chemische Haftfestigkeit der keramischen Verblendung infolge der Ionenmodifikation erhöht.

Nach einer "Ruhezeit" von einer Viertelstunde zur Passivierung der Titanoberfläche erfolgte die Reinigung mit dem Dampfstrahlgerät.

Beispiel 2: Oberflächenmodifikation durch die Ionenimplantation

Die beiden bezüglich der mechanischen Oberflächenbearbeitung unterschiedlichen Chargen wurden geteilt und eine Hälfte durch die Ionenimplantation modifiziert. Als Implantationsanlage diente der LINEAR ION IMPLANTER LION 6000, hergestellt von der Firma LEYBOLD AG Hanau.

Die Ionenimplantation erfolgte mit Siliziumionen 4, wobei davon ausgegangen wurde, daß in der Oberfläche 1 der Probenkörper die Durchdringungszone zwischen den Titanatomen 5 und den Siliziumionen 4 entsteht. Mit der entstehenden Titan-Silizium-Schicht 2 werden durch deren chemische Eigenschaften die Reaktionen der Oberfläche 1 des Titans blockiert. (Fig. 1)

Die Ionendosis betrug $3 \times 10^{17}$ Atome/$cm^2$ und die Ionenenergie 150 KeV.

Der bevorzugte Bereich ist für die Ionendosis zwischen $1 \times 10^{12}$ bis $1 \times 10^{18}$ Atome/$cm^2$ und für die Ionenenergie von 30 bis 400 KeV.

Die nicht durch Siliziumimplantation modifizierten Prüfkörper dienten als Vergleichscharge zur Überprüfung der Auswirkungen der Ionenimplantation auf den Titan-Keramik-Haftverbund.

Beispiel 3: Die verwendeten Titan-Keramikmassen

Für die Verblendung der Probekörper wurden folgende spezielle Titan-Keramikmassen verwendet:

- VITA TITANKERAMIK, hergestellt durch die Firma VITA ZAHN-FABRIK/Bad Säckingen,

- TIBOND, hergestellt durch die Firma DE TREY/DENTSPLY/ Dreieich.

In der Tabelle 3 ist die Zusammensetzung der Titan-Keramikmasse "VITA TITANKERAMIK" in Masse-% dargelegt:

Tabelle 3:

| VITA TITANKERAMIK Zusammensetzung in Masse-% | | | |
|---|---|---|---|
| | Bonder | Opaker | Dentin/Schmelz |
| $SiO_2$ | 61,2-64,0 | 59,5-62,1 | 67,1-69,0 |
| $Al_2O_3$ | 6,2-6,6 | 7,3-7,7 | 7,2-7,5 |
| $K_2O$ | 3,2-3,7 | 7,1-7,6 | 8,2-8,7 |
| $Na_2O$ | 5,1-5,4 | 5,3-5,7 | 6,0-6,3 |
| CaO | 4,5-5,0 | 1,0-1,3 | 1,1-1,4 |
| $B_2O_3$ | --- | 6,1-6,9 | 8,0-8,4 |
| BaO | 2,0-2,3 | 0,1-0,3 | --- |
| $SnO_2$ | 2,1-2,5 | 2,1-2,7 | --- |
| MgO | 0,5-0,8 | 8,0-8,4 | --- |
| $TiO_2$ | --- | 8,0-8,4 | --- |

In der folgenden Tabelle 4 sind die Brenndaten, wie sie von der Firma VITA ZAHNFABRIK, Bad Säckingen in der Verarbeitungsanleitung genannt sind und wie sie während der Brennzyklen verwendet wurden, genannt.

Tabelle 4:

| VITA TITANKERAMIK Brenndaten | | | | | | | |
|---|---|---|---|---|---|---|---|
| VITA TITANKERAMIK | Bereitschaftstemp.°C | Brenntemp.°C | Vortrocken zeit | Aufheizzeit | Haltezeit | Vakuum | Langzeitabkühlung |
| Bonder | 600 | 800 | 6 min | 1 min | 7 min | 7 min | + |
| Grund/Opk | 400 | 790 | 2 min | 3 min | 1 min | 4 min | + |
| Dentin | 400 | 770 | 6 min | 7 min | 1 min | 8 min | + |
| Korrektur | 400 | 770 | 6 min | 7 min | 1 min | 8 min | + |
| Glanz | 400 | 770 | 3 min | 3 min | 2 min | -- | + |

In der Tabelle 5 ist die Zusammensetzung der Titan-Keramikmasse "TIBOND" dargelegt:

Tabelle 5:

| TIBOND Zusammensetzung in Masse-% | | | |
|---|---|---|---|
| | Bonder | Opaker | Dentin/Schmelz |
| $SiO_2$ | 63-65 | 44,1-45,5 | 64-66 |
| $Al_2O_3$ | 6-7 | 8,4-9,1 | 12-13 |
| $K_2O$ | 7-8 | 6,3-7,0 | 7-9 |
| $Na_2O$ | 5-6 | 4,2-4,9 | 5-6 |
| $Li_2O$ | 2-3 | 0,7-1,4 | 1-2 |
| CaO | 3-4 | 1,1-2,1 | 1-2 |
| $B_2O_3$ | 10-11 | 2,8-3,5 | 5-7 |

In der folgenden Tabelle 6 sind die Brenndaten, wie sie von der Firma DE TREY DENTSPLY/Dreieich in der Gebrauchsinformation und wie sie während der Brennzyklen verwendet wurden, genannt:

Tabelle 6:

| TIBOND Brenndaten | | | | | | |
|---|---|---|---|---|---|---|
| TIBOND | Bereitschaftstemp °C | Brenntemperatur °C | Vortrokkenzeit | Aufheizzeit | Haltezeit | Vakuum |
| Bonder | 650 | 780 | 2 min | 2 min | 3 min | 1 min |
| Grund/Opk | 650 | 760 | 3 min | 3 min | 3 min | 2 min |
| Dentin | 650 | 750 | 6 min | 3 min | 2 min | 4 min |
| Korrektur | 650 | 750 | 6 min | 3 min | 2 min | 4 min |
| Glanz | 650 | 740 | 3 min | 3 min | 2 min | --- |

Beispiel 4: Die Prüfung der Verbundfestigkeiten

Zur Untersuchung der Haftfestigkeit keramischer Verblendungen von Titangerüsten ist ein bruchmechanischer Test zur Bestimmung der Verbundfestigkeit geeignet, der bei möglichst exakter Reproduzierbarkeit der Prüfkörperperherstellung mit geringem technischen Aufwand eine minimale Toleranz der "Streuung" der Prüfergebnisse aufweist.

Zusätzlich sollen die Prüfkörpergestaltung und Prüfanordnung eine genaue quantitative Beurteilung der Verbundfestigkeit bzw. Haftkraft erlauben. Der Schertest nach SCHMITZ-SCHULMEYER erfüllt diese Anordnungen und ermöglicht darüber hinaus durch die Versuchsanordnung und -durchführung unter Ausschaltung von Nebeneinflüssen, wie Radialspannungen und Biegemomenten, in der keramischen Verblendung sowie plastischen oder elastischen Verformung innerhalb der Metallgerüste eine exakte Aussage über die tatsächliche Haftfestigkeit zwischen Metall und Keramik unter technischen Gesichtspunkten.

Bei der Versuchsdurchführung wird eine modifizierte Ausführung des Schertestes nach SCHMITZ-SCHULMEYER angewandt.

Die Prüfkörper für den Schertest haben, wie bereits dargelegt, die Form eines quadratischen Würfels mit einer Kantenlänge von 5,9 mm. Der Probekörper aus Titan wird auf einer Würfelfläche mit der keramischen Masse verblendet, wobei eine Hälfte der Würfelfläche bedeckt, die andere Hälfte der Würfelfläche jedoch nicht bedeckt ist.

Im Gegensatz zu dem von SCHMITZ-SCHULMEYER angegebenen plangeschliffenen Laststempel hat der verwendete Laststempel die Form einer, in einem Winkel von 45° angeschliffenen, Druckfinne. Diese Laststempelgeometrie ermöglicht es, den Kraftansatzpunkt des Laststempels in problemlos reproduzierbarer Lage sehr nahe (< 1 mm) an dem Übergang Keramik - Metall zu positionieren. Durch dieses Vorgehen wird ein nicht vollständig zu vermeidendes Biegemoment möglichst klein gehalten und vermieden, den Laststempel unbeabsichtigt so weit von der Grenzfläche Bonder/Grundmasse entfernt zu positionieren, daß die Haftfestigkeit zwischen Bonder und Grundmasse bzw. deren Biegefestigkeit zu einem das Meßergebnis beeinflussenden Faktor wird.

Die mit der keramischen Verblendung versehenen Würfel werden in einem speziellen Scherwerkzeug fixiert und in einer mechanischen Druck-Biegeprüfmaschine geprüft, wobei es möglich ist, die Prüfkörper stets in die gleiche Lage zum Laststempel zu bringen. Der Laststempel wird möglichst nahe an der Metallkante positioniert und die keramische Masse durch den Laststempel mit einem Vorschub von 1,0 mm/min bis zum völligen Abscheren der Grundmasse belastet.

Beispiel 5: REM-Rückstrahlbilder und halbquantitative EDAX-Analysen

REM-Rückstrahlbilder und halbquantitative EDAX-Analysen wurden von vorbereiteten Prüfkörpern an typischen Stellen des Verbundes angefertigt.

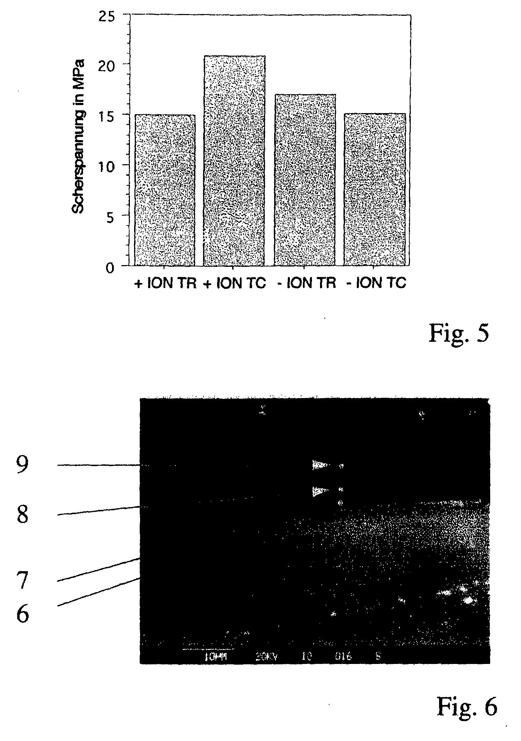

In Fig. 6 ist die Anordnung der Meßpunkte der EDAX-Analyse gezeigt. Dabei liegt der Meßpunkt 6 ca. 2 μm innerhalb des Titankörpers und der Meßpunkt 7 auf der sichtbaren Titan-Keramik-Grenze. Die Meßpunkte 8; 9 befinden sich 2 μm und 5 μm in der Keramik-, respektive in der Bonder-Masse. Der Bereich der Titan-Keramik-Kontaktzone wurde zusätzlich auf das Vorhandensein von Kavernen mit hohem Aluminiumanteil untersucht. Die Ergebnisse der punktuellen EDAX-Untersuchungen der REM-Schnittbilder mit den angefertigten Elektronenrückstrahlbildern wurden zu den aus dem Scherversuch stammenden Haftfestigkeitswerten in Beziehung gesetzt.

Beispiel 6: Herstellung der Probekörper für den Schertest nach SCHMITZ-SCHULMEYER

Die Probekörper wurden entsprechend ihrer Vorbehandlung bzw. Oberflächenkonditionierung in unterschiedlichen Prüfserien eingeteilt, die sich den beiden übergeordneten Vergleichsgruppen Ionen implantiertes / nicht Ionen implantiertes Titan zuordnen lassen (Tabelle 7).

Tabelle 7:

| Titan-Keramik-Kombination | | | |
|---|---|---|---|
| Titan, Ionen implantiert | | Titan, nicht Ionen implantiert | |
| $\alpha$-Al$_2$O$_3$-gestrahlt | nicht $\alpha$-Al$_2$O$_3$-gestrahlt | $\alpha$-Al$_2$O$_3$-gestrahlt | nicht $\alpha$-Al$_2$O$_3$-gestrahlt |
| TIBOND, TR/TC | | | |
| VITA/TITANKERAMIK TR/TC | | | |

Zur Durchführung der keramischen Verblendung stand ein Vakuum-Keramikofen VACUMAT 300 der Firma VITA zur Verfügung. Es handelt sich dabei um einen mikroprozessorgesteuerten, frei programmierbaren und voll automatisch arbeitenden Keramikofen, der sämtliche in den Tabellen 4 und 6 genannte Brenndaten ermöglicht.

Die keramischen Massen wurden nach Dampfstrahlreinigung der zu verblendenden Prüfkörperfläche, den Herstellerangaben folgend, aufgetragen und gebrannt, wobei bei allen Prüfkörpern vier Brennzyklen durchlaufen wurden.

1. Zyklus: Bonder und/oder Washbrand-Masse
2. Zyklus: Grundmassebrand
3. Zyklus: Dentinbrand
4. Zyklus: Glanzbrand

Die Probekörper für den Schertest wurden zwei unterschiedlichen Lagerungsbedingungen unterzogen.

- Eine Hälfte der Probekörper wurde nach 24 Stunden Trockenlagerung (TR) bei Normalklima nach DIN 50014-23/50-2 getestet.

- Die andere Hälfte der Probekörper wurde nach keramischer Verblendung einer künstlichen Alterung durch 5000 Temperaturlastwechselzyklen (Thermocycling, TC) im Wasserbad zugeführt. Der Temperaturunterschied betrug 50 °C (+5 °C <--> +55 °C), die Haltezeit der jeweiligen Temperaturstufe 60 Sekunden und die Überleitungszeit 5 Sekunden. Unverzüglich nach Abschluß der künstlichen Alterung wurde die Prüfung der Haftfestigkeit vorgenommen.

Je Untersuchungsreihe wurden 12 Prüfkörper erstellt und gegebenenfalls nach dem Zufallsverfahren 10 Prüfkörper zur Auswertung ausgewählt.

**EP 0 910 684 B1**

Beispiel 7: Herstellung der Probekörper für die REM-EDAX Analyse

Für die REM-EDAX Analyse wurden Probeplättchen der Maße 5 x 10 x 3 mm angefertigt.

Von diesen Plättchen wurde je ein Exemplar einer Untersuchungsserie zugeordnet und die entsprechenden Oberflächen-Konditionierungen und -bearbeitungen sowie keramischen Verblendungen und die Lagen, nämlich Trockenlagerung und Thermocycling, durchgeführt.

Anschließend wurden die Probekörper in ein Kunstharz auf Polyesterbasis der Marke AKEMI-TRANSPARENT, hergestellt durch die Firma JEAN WIRTZ, eingebettet. Anschließend wurden sie nach dem Aushärten unter ausreichender Kühlung, längs der 10 mm langen Kante des Probeplättchens, mittig zersägt.

Im darauffolgenden Arbeitsgang wurden die Proben mittels Schleifscheiben eines Durchmessers von 25 cm verschiedener Korngrößen (400 μm, 600 μm, 1000 μm, 1200 μm) mit dem Schleifgerät (Typ TF 250 der Firma JEAN WIRTZ) bearbeitet. Die abschließende Hochglanzpolitur erfolgte mit DIAPAST, einer Diamantpolierpaste mit einer Korngröße von 6 und 3 μm und dem Diamantschmiermittel DIALUB SW, hergestellt von der Firma JEAN WIRTZ.

Um die so angefertigten Proben für die REM-Untersuchung leitfähig zu machen, wurden sie in einem Gerät Typ SCD 040, hergestellt von der Firma BALZERS UNION, besputtert.

Die Durchführung der rasterelektronenmikroskopischen Untersuchungen erfolgte an einem Elektronenmikroskop 150 MK2, hergestellt von der Firma CAMBRIDGE-STERECAN.

Beispiel 8: Versuchsdurchführung des Schertests

Die Schertests nach SCHMITZ-SCHULMEYER wurden mit einer ZWICK-Universalprüfmaschine vom Typ 1435 durchgeführt. Dabei kam eine Kraftmeßdose von 1000 N zur Anwendung. Die Prüfkörper wurden in der Einspannvorrichtung des Scherwerkzeuges mit Hilfe der Feststellschrauben in stets gleicher Lage zum Laststempel fixiert. Der Laststempel wurde an die Keramikoberfläche herangefahren und die Keramik mit einer Vorschubgeschwindigkeit von 1 mm/min bis zum vollständigen Abscheren belastet.

Die Kräfte, die zum Abscheren der Keramik führen, wurden in NEWTON gemessen und von einem Bandschreibegerät auf Millimeterpapier aufgezeichnet. Die Scherspannung errechnet sich aus der gemessenen, aufgewendeten Kraft und der Oberfläche, die mit keramischer Masse verblendet war. Dementsprechend wird die Oberfläche nach dem Scherversuch mit Hilfe eines Stereomikroskopes mit eingebautem Okularmikrometer vermessen. Die als Maß für die Haftfestigkeit dienende Scherspannung resultiert aus dem Quotienten von Kraft pro Oberfläche nach der Formel:

$$\text{Scherspannung } \frac{N}{mm^2} = \frac{\text{Kraft F [N]}}{\text{Fläche [mm}^2\text{]}}$$

Die Ermittlung der Scherspannung in MPa (Megapascal) führte ein Computer durch, der direkt an die ZWICK-Universalprüfmaschine angeschlossen war.

Beispiel 9: Ergebnisse des Schertests nach SCHMITZ-SCHULMEYER

Versuch 9.1: Titan $\alpha$-Al$_2$O$_3$-gestrahlt/TIBOND

In der Tabelle 8 sind die Absolutwerte der Einzelmessungen der Probekörper mit

Keramische Masse:     TIBOND
Titanoberfläche:          $\alpha$-Al$_2$O$_3$-gestrahlt, Ionen implantiert

gezeigt.

Tabelle 8:

| n | TR $\tau$/Mpa | TC $\tau$/MPa |
|---|---|---|
| 1 | 12,9 | 9,1 |
| 2 | 15,6 | 16,4 |
| 3 | 13,0 | 14,6 |

Tabelle 8: (fortgesetzt)

| n | TR $\tau$/Mpa | TC $\tau$/MPa |
|---|---|---|
| 4 | 10,5 | 8,2 |
| 5 | 11,8 | 20,2 |
| 6 | 10,2 | 15,3 |
| 7 | 17,1 | 17,0 |
| 8 | 16,3 | 9,7 |
| 9 | 20,8 | 6,8 |
| 10 | 9,2 | 8,4 |

Hierzu bedeuten:

n: Nummer des Prüfkörpers
TR: Trockenlagerung
TC: Thermocycling
$\tau$: Scherspannung in MPa

Die Bezeichnungen werden ebenfalls in den nachstehenden Tabellen verwendet.
In der Tabelle 9 sind die Absolutwerte der Einzelmessungen der Probekörper für

keramische Masse: TIBOND
Titanoberfläche: $\alpha$-$Al_2O_3$-gestrahlt, nicht Ionen implantiert

gezeigt.

Tabelle 9:

| n | TR $\tau$/Mpa | TC $\tau$/MPa |
|---|---|---|
| 1 | 17,1 | 22,6 |
| 2 | 20,2 | 22,6 |
| 3 | 14,0 | 15,5 |
| 4 | 18,3 | 14,4 |
| 5 | 23,4 | 23,9 |
| 6 | 22,4 | 18,2 |
| 7 | 23,2 | 18,9 |
| 8 | 17,7 | 17,2 |
| 9 | 15,2 | 10,7 |
| 10 | 12,0 | 14,2 |

Hierzu ermöglicht die graphische Darstellung in der Fig. 2 einen Überblick über die Medianwerte für das Verbundsystem zwischen $\alpha$-$Al_2O_3$-gestrahltem Titan und der Titan-Keramikmasse TIBOND sowie den Vergleich zwischen Ionen implantierten und nicht Ionen implantierten Oberflächen in Abhängigkeit von den Lagerungsbedingungen.
Der Medianwert der Haftfestigkeit der Ionen implantierten Probekörperserien beträgt nach Trockenlagerung 18 MPa und fällt nach Thermocycling auf 17,7 MPa ab. Im Gegensatz dazu fällt die Haftfestigkeit bei den nicht Ionen implantierten Vergleichsserien von 13 MPa auf 12,2 MPa.
Der prozentuale Rückgang der Haftfestigkeit fällt nach Ionenimplantation mit 1,7 % gegenüber 6,2 % Haftfestigkeitsverlust ohne Ionenimplantation deutlich geringer aus.
Der direkte Vergleich der Ionen implantierten und der nicht Ionen implantierten Serien unter gleichen Lagerungsbedingungen zeigt nach Ionenimplantation bei Trockenlagerung um 27,8 % und nach Thermocycling um 31,1 % höhere Werte der Haftfestigkeit.

Versuch 9.2 : Titan $\alpha$-Al$_2$O$_3$-gestrahlt/VITA TITANKERAMIK

In der Tabelle 10 sind die Absolutwerte der Einzelmessungen der Probekörper für

keramische Masse: VITA TITANKERAMIK
Titanoberfläche: $\alpha$-Al$_2$O$_3$-gestrahlt, Ionen implantiert

gezeigt.

Tabelle 10 :

| n | TR $\tau$/Mpa | TC $\tau$/MPa |
|---|---|---|
| 1 | 19,2 | 25,0 |
| 2 | 22,0 | 22,8 |
| 3 | 19,5 | 21,1 |
| 4 | 25,9 | 25,6 |
| 5 | 24,7 | 21,0 |
| 6 | 17,1 | 22,1 |
| 7 | 27,3 | 25,2 |
| 8 | 27,3 | 21,3 |
| 9 | 22,0 | 9,9 |
| 10 | 19,4 | 16,3 |

In der Tabelle 11 sind die Absolutwerte der Einzelmessungen der Probekörper für

keramische Masse: VITA TITANKERAMIK
Titanoberfläche : $\alpha$-Al$_2$O$_3$-gestrahlt, nicht Ionen implantiert

gezeigt.

Tabelle 11:

| n | TR $\tau$/MPa | TC $\tau$/MPa |
|---|---|---|
| 1 | 23,7 | 18,7 |
| 2 | 32,5 | 20,2 |
| 3 | 18,1 | 9,8 |
| 4 | 24,9 | 15,6 |
| 5 | 42,9 | 13,8 |
| 6 | 31,3 | 18,5 |
| 7 | 16,1 | 21,4 |
| 8 | 24,1 | 8,2 |
| 9 | 21,0 | 19,6 |
| 10 | 20,4 | 17,9 |

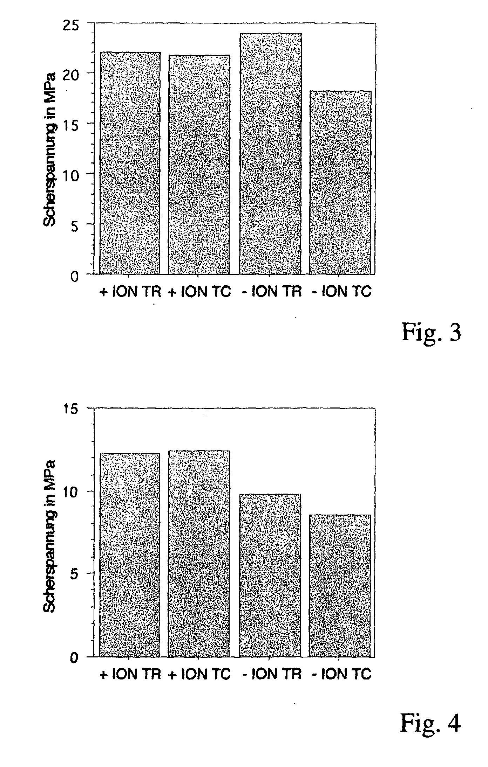

Die graphische Darstellung in der Fig. 3 gibt einen Überblick über die Medianwerte der Verbundfestigkeit in MPa auf der Grundlage der ermittelten Meßwerte.
Für das Ionen implantierte Titan ergab sich nach Trockenlagerung ein Wert von 22 MPa, und nach Thermocycling wurden 21,7 MPa ermittelt.
Der Wert für das nicht Ionen implantierte Titan beträgt nach Trockenlagerung 23,9 MPa und nach Thermocycling 18,2 MPa.

Der Vergleich der Rückgänge der Haftfestigkeiten in Prozent zeigt für die Ionen implantierten Serien einen Wert von 1,4 % gegenüber 17,3 % bei nicht Ionen implantierter Titanoberfläche. Der Vergleich gleicher Lagerungsbedingungen fällt unterschiedlich aus.

Unter Trockenlagerung liegt die Haftfestigkeit der Ionen implantierten Serie 7,9 % unterhalb der nicht Ionen implantierten Serie. Nach Thermocycling fällt der Wert der nicht Ionen implantierten Serie um 16,1 % geringer aus als bei Ionen implantiertem Titan.

Versuch 9.3: Titan nicht $\alpha$-Al$_2$O$_3$-gestrahlt/TIBOND

In der Tabelle 12 sind die Absolutwerte der Einzelmessungen der Probekörper mit

keramische Masse: TIBOND
Titanoberfläche: nicht $\alpha$-Al$_2$O$_3$-gestrahlt, Ionen implantiert

gezeigt.

Tabelle 12:

| n | TR $\tau$/Mpa | TC $\tau$/MPa |
|---|---|---|
| 1 | 9,0 | 14,4 |
| 2 | 14,7 | 9,8 |
| 3 | 7,8 | 15,6 |
| 4 | 12,0 | 15,0 |
| 5 | 10,2 | 7,8 |
| 6 | 15,0 | 12,8 |
| 7 | 14,9 | 9,8 |
| 8 | 11,5 | 13,5 |
| 9 | 12,6 | 11,6 |
| 10 | 13,8 | 12,0 |

In der Tabelle 13 sind die Absolutwerte der Einzelmessungen der Probekörper für

keramische Masse: TIBOND
Titanoberfläche: nicht $\alpha$-Al$_2$O$_3$-gestrahlt, nicht Ionen implantiert

gezeigt.

Tabelle 13:

| n | TR $\tau$/MPa | TC $\tau$/MPa |
|---|---|---|
| 1 | 14,4 | 7,5 |
| 2 | 10,2 | 12,0 |
| 3 | 9,7 | 14,3 |
| 4 | 6,6 | 9,6 |
| 5 | 12,1 | 6,9 |
| 6 | 10,8 | 7,1 |
| 7 | 8,2 | 8,8 |
| 8 | 9,9 | 10,7 |
| 9 | 9,1 | 7,7 |
| 10 | 8,7 | 8,3 |

Die graphische Darstellung in der Fig. 4 zeigt den Überblick über die Medianwerte der Verbundfestigkeit in MPa auf der Grundlage der Meßwerte in den Tabellen 12 und 13.

Das durch Ionenimplantation modifizierte Titan dieser Probekörper zeigte nach Trockenlagerung eine Haftfestigkeit von 12,3 MPa, die nach Thermocycling eine Erhöhung auf 12,4 MPa erfuhr.

Ohne Ionenimplantation lagen die Werte bei 9,8 MPa für Trokkenlagerung und bei 8,6 MPa nach Thermocycling.

Der Vergleich der Veränderung der Haftfestigkeit mit und ohne Ionenimplantation weist mit Modifikation eine geringfügige Erhöhung um 0,8 % aus, wohingegen ohne Modifikation ein Verlust von 12,2 % auftritt.

Bei Trockenlagerung fiel der Wert für nicht Ionen implantiertes Titan um 20,3 % niedriger als bei Ionenimplantation aus. Der prozentuale Vergleich nach Thermocycling zeigt für nicht Ionen implantiertes Titan 30,7 % geringere Werte als für Ionen modifiziertes Titan.

Versuch 9.4: Titan nicht $\alpha$-Al$_2$O$_3$-gestrahlt/VITA TITAN-KERAMIK

In der Tabelle 14 sind die Absolutwerte der Einzelmessungen der Probekörper mit

| keramische Masse: | VITA TITANKERAMIK |
|---|---|
| Titanoberfläche: | nicht $\alpha$-Al$_2$O$_3$-gestrahlt, Ionen implantiert gezeigt. |

Tabelle 14:

| n | TR $\tau$/Mpa | TC $\tau$/MPa |
|---|---|---|
| 1 | 14,3 | 12,4 |
| 2 | 17,9 | 10,2 |
| 3 | 24,5 | 17,9 |
| 4 | 12,8 | 27,6 |
| 5 | 16,3 | 22,8 |
| 6 | 8,8 | 22,4 |
| 7 | 22,1 | 16,1 |
| 8 | 15,7 | 19,4 |
| 9 | 13,0 | 22,7 |
| 10 | 13,6 | 25,9 |

In der Tabelle 15 sind die Absolutwerte der Einzelmessungen der Probekörper für

| keramische Masse: | VITA TITANKERAMIK |
|---|---|
| Titanoberfläche: | nicht $\alpha$-Al$_2$O$_3$-gestrahlt, nicht Ionen implantiert |

gezeigt.

Tabelle 15:

| n | TR $\tau$/MPa | TC $\tau$/MPa |
|---|---|---|
| 1 | 13,0 | 17,8 |
| 2 | 18,3 | 14,8 |
| 3 | 17,4 | 21,0 |
| 4 | 16,6 | 10,9 |
| 5 | 12,8 | 7,1 |
| 6 | 18,6 | 16,6 |
| 7 | 20,5 | 19,6 |

Tabelle 15: (fortgesetzt)

| n | TR $\tau$/MPa | TC $\tau$/MPa |
|---|---|---|
| 8 | 10,0 | 15,2 |
| 9 | 19,7 | 13,3 |
| 10 | 14,8 | 15,1 |

Die graphische Darstellung in der Fig. 5 zeigt den Überblick über die Medianwerte der Verbundfähigkeit in MPa auf der Grundlage der Meßwerte in den Tabellen 14 und 15.

Die Haftfestigkeitswerte der Ionen implantierten Probekörper liegen nach Thermocycling mit 20,9 MPa 39 % oberhalb der Werte von 15 MPa nach Thermocycling. Die Haftfestigkeit der nicht-modifizierten Vergleichsserie liegt nach Trockenlagerung bei 17 MPa und fällt um 10,6 % auf 15,2 MPa nach Thermocycling ab.

Bei Trockenlagerung liegt der Wert für ionenstrahlmodifiziertes Titan 11,8 % unterhalb des Wertes für nicht modifiziertes Titan. Nach Thermocycling kehren sich die Verhältnisse um, und die Werte der nicht modifizierten Serie unterschreiten die der modifizierten Serie um 27,3 %.

Beispiel 10: Ergebnisse der EDAX- und REM-Untersuchungen

Die Anordnung der Meßpunkte der EDAX-Analysen ist in Fig. 6 gezeigt und in Beispiel 5 "REM-Rückstrahlbild und halbquantitative EDAX-Analysen" bereits beschrieben.

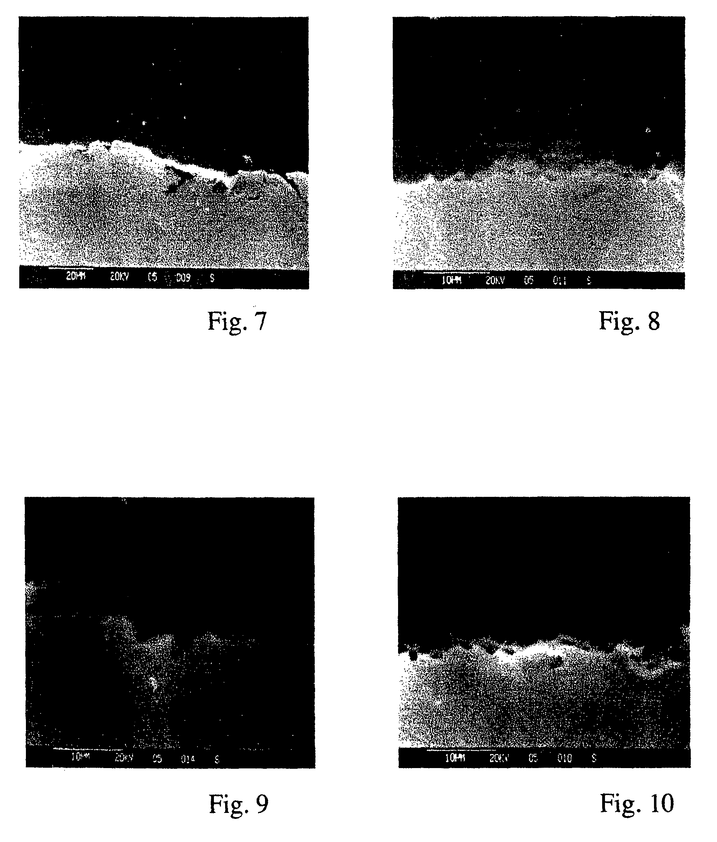

Die REM-Aufnahmen, wie sie in den Fig. 7 bis 14 dargestellt sind, zeigen bei 2000-, 3200- bzw. 3300-facher Vergrößerung die Kontaktzone der Übergangs Titan - Keramik. Das Titan erscheint als unstrukturierte hellgraue Fläche. Die Glasmatrix der Keramik stellt sich im Vergleich dazu in einem dunkleren, blaßblauen Farbton dar. Metallische Oxide sind an ihrer dunkelgrauen bis anthrazitähnlichen Farbe zu erkennen. Einlagerungen von schweren Metallionen mit großer Dichte erscheinen hell bis weiß. Die Darstellung der Ergebnisse erfolgt anhand der dem Thermocycling unterzogenen Prüfkörper, und in den nachfolgenden Tabellen erfolgen die Angaben bei den wichtigsten Elementen (Ti, Si, Al).

Der direkte Vergleich der mit der Ionenimplantation durch Silizium modifizierten Probekörper mit den nicht modifizierten Probekörpern gleicher mechanischer Oberflächenbehandlung und gleichem keramischen Verblendmaterial ermöglicht die anschauliche Darstellung der durch die Modifikation induzierten Veränderungen der Titan-Keramik-Kontaktzone.

Der in den folgenden Tabellen als Rest deklarierte Anteil bezeichnet die im EDAX-Verfahren nicht identifizierbaren Elemente.

Versuch 10.1: Titan $\alpha$-Al$_2$O$_3$-gestrahlt/TIBOND, TC

In den Fig. 7 und 8 sind die REM-Aufnahmen der Kombinationen Titan $\alpha$-Al$_2$O$_3$-gestrahlt/TIBOND, mit Thermocycling TC behandelt, gezeigt.

Die Fig. 7 und 8 zeigen die infolge der Korundstrahlung aufgerauhte Titanoberfläche mit guter Verzahnung zwischen dem Metall und der Keramik, was auf eine gute Benetzung der Oberfläche des Titans durch den Bonder schließen läßt. Der Bonder des Ionen implantierten Probekörpers zeigt geringfügige Porositäten.

Ohne Ionenimplantation (Fig. 8) zeigt sich eine deutlich sichtbare, schuppig-kristalline Zwischenschicht oberhalb der Titanoberfläche, die eine Breite von ca. 6 $\mu$m aufweist, während mit Ionenimplantation (Fig. 7) kristalline Schuppen praktisch nicht ausgemacht werden können. Dementsprechend hoch wurden die Titan- und Siliziumkonzentrationen am Meßpunkt 8 (Fig. 6) ohne Ionenimplantation gemessen. Bei beiden Fig. 7 und 8 sind Kavernen sichtbar, in denen die gemessenen Aluminiumkonzentrationen hohe Werte annehmen (32 % bei Ionenimplantation und 92,2 % ohne Ionenimplantation).

Die gemessenen Werte der Konzentrationen in Prozent für die angegebenen Elemente sind der Tabelle 16 zu entnehmen.

Tabelle 16:

| Ergebnisse der EDAX-Analyse für die Kombination Titan $\alpha$-Al$_2$O$_3$-gestrahlt/TIBOND, TC | | | | | | | |
|---|---|---|---|---|---|---|---|
| | 2 µm im Metall (Meßpunkt 6) | | Kontaktzone (Meßpunkt 7) | | 2 µm in der Keramik (Meßpunkt 8) | | 5 µm in der Keramik (Meßpunkt 9) | |
| | + ION | - ION | + ION | - ION | + ION | - ION | + ION | - ION |
| Ti | 83,4 | 98,9 | 26,5 | 78,3 | 2,7 | 13,7 | 0,8 | 0,9 |
| Si | 6,4 | 0,0 | 20,3 | 2,3 | 40,0 | 51,1 | 34,4 | 35,1 |
| Al | 4,2 | 0,0 | 5,6 | 4,4 | 11,0 | 3,3 | 3,5 | 4,1 |
| Rest | 3,3 | 1,1 | 24,8 | 15,3 | 23,3 | 31,1 | 48,1 | 45,6 |

Versuch 10.2.: Titan $\alpha$-Al$_2$O$_3$-gestrahlt/VITA TITANKERAMIK, TC

Die REM-Aufnahme ist in den Fig. 9 und 10 gezeigt.

Die durch Korundstrahl aufgerauhte Titanoberfläche zeigt ähnlich wie bei den entsprechenden TIBOND Vergleichsserien eine gute Benetzung zwischen Bonder und Titan. Eine schuppig-kristalline Zwischenschicht ist bei dem nicht Silizium implantierten Probekörper deutlich sichtbar (Fig. 10), während die durch Ionenimplantation konditionierte Kontaktzone keine vergleichbaren Strukturen erkennen läßt (Fig. 9). Die EDAX-Analysen bestätigen den optischen Eindruck, wobei vor allem die Elementverteilung am Meßpunkt 8 (Fig. 6) von Bedeutung ist. Mit Ionenimplantation ist an dieser Stelle eine bedeutend geringere Titankonzentration als ohne Ionenimplantation nachzuweisen. Auch bei dieser Kombination von Titan, Keramik und mechanischer Oberflächenbearbeitung sind sichtbare Kavernen mit einer hohen Aluminiumkonzentration von 66,4 % ohne Ionenimplantation und 71 % mit Ionenimplantation vorhanden.

Der Tabelle 17 sind die gemessenen Werte zu entnehmen.

Tabelle 17:

| Ergebnisse der EDAX-Analyse für die Kombination Titan $\alpha$-Al$_2$O$_3$-gestrahlt/VITA TITANKERAMIK, TC | | | | | | | |
|---|---|---|---|---|---|---|---|
| | 2 µm im Metall (Meßpunkt 6) | | Kontaktzone (Meßpunkt 7) | | 2 µm in der Keramik (Meßpunkt 8) | | 5 µm in der Keramik (Meßpunkt 9) | |
| | + ION | - ION | + ION | - ION | + ION | - ION | + ION | - ION |
| Ti | 100,00 | 100,0 | 49,7 | 61,8 | 1,5 | 9,6 | 0,9 | 1,4 |
| Si | 0,0 | 0,0 | 17,0 | 9,7 | 33,3 | 48,6 | 30,9 | 32,7 |
| Al | 0,0 | 0,0 | 4,4 | 8,6 | 3,2 | 5,4 | 7,8 | 4,6 |
| Rest | 0,0 | 0,0 | 23,4 | 17,2 | 46,8 | 30,2 | 42,8 | 48,7 |

Versuch 10.3.: Titan nicht $\alpha$-Al$_2$O$_3$-gestrahlt/TIBOND, TC

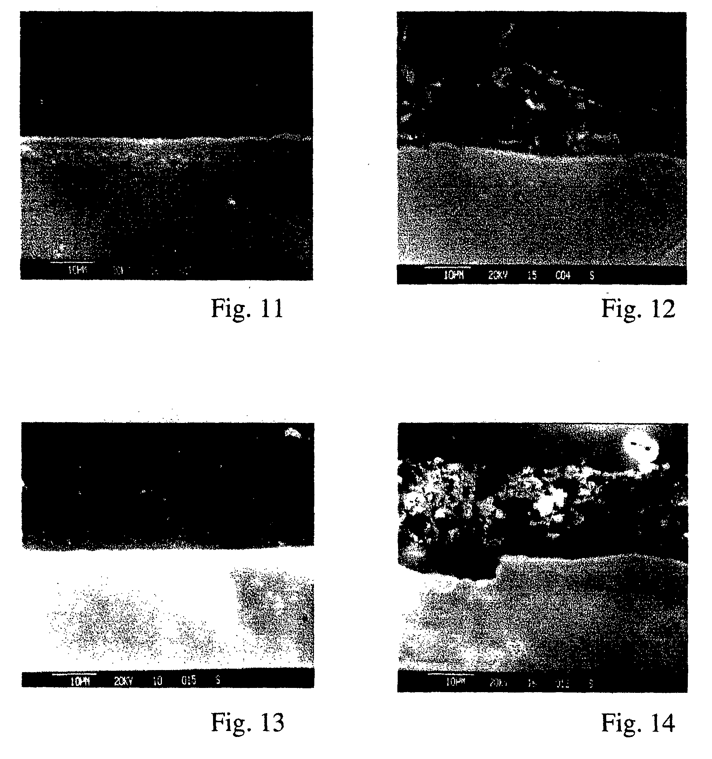

Die REM-Aufnahmen sind in den Fig. 11 und 12 gezeigt.

Im Vergleich zu den mit Korund gestrahlten Probekörpern fällt die ebenmäßige Metalloberfläche auf. Die Benetzung mit dem Bonder bleibt davon unbeeinflußt.

Die nicht durch die Ionenstrahl modifizierte Titan-Keramik-Kontaktzone zeigt deutliche, schuppig-kristalline Strukturen, die sich relativ weit in die Bonderschicht ausdehnen. Bei dieser EDAX-Analyse ist im Meßpunkt 8 ohne Ionenimplantation eine außerordentlich hohe Titankonzentration nachweisbar, während die Siliziumkonzentration geringere Werte annimmt. In Kontrast zu den $\alpha$-Al$_2$O$_3$ -gestrahlten Probekörpern sind die nicht identifizierbaren Restelemente an der Titankante (Meßpunkt 7) niedriger konzentriert.

Tabelle 18:

| Ergebnisse der EDAX-Analyse für die Kombination Titan nicht α-Al$_2$O$_3$-gestrahlt/TIBOND, TC | | | | | | | |
|---|---|---|---|---|---|---|---|
| | 2 µm im Metall (Meßpunkt 6) | | Kontaktzone (Meßpunkt 7) | | 2 µm in der Keramik (Meßpunkt 8) | | 5 µm in der Keramik (Meßpunkt 9) |
| | + ION | - ION | + ION | - ION | + ION | - ION | + ION | - ION |
| Ti | 98,8 | 100,0 | 88,8 | 81,2 | 1,2 | 41,5 | 0,6 | 1,7 |
| Si | 0,0 | 0,0 | 5,2 | 3,4 | 33,7 | 14,5 | 38,2 | 31,7 |
| Al | 0,0 | 0,0 | 1,2 | 0,7 | 4,5 | 2,2 | 3,2 | 5,9 |
| Rest | 1,2 | 0,0 | 1,8 | 11,6 | 46,3 | 33,1 | 44,7 | 47,1 |

Versuch 10.4.: Titan nicht α-Al$_2$O$_3$-gestrahlt/VITA TITAN-KERAMIK, TC

Die REM-Aufnahmen sind in den Fig. 13 und 14 gezeigt.

Auch bei diesem Titan-Keramik-Haftverbund sind deutliche Unterschiede sichtbar. Den vorangestellten, vergleichenden Betrachtungen entsprechend, weist die Bonderschicht auf dem nicht durch Ionenbeschuß konditionierten Titan kristalline Strukturen auf, die durch ein körniges Erscheinungsbild gekennzeichnet sind. Auch in diesem Fall durchsetzen diese Strukturen die gesamte Bonderschicht und weisen am Meßpunkt 8 hohe Titananteile von 12 % auf. Im Gegensatz dazu ist beim Ionen implantierten Titan die Bonderschicht homogen und nicht strukturiert. Dementsprechend sind 2 µm oberhalb der Metallkante (Meßpunkt 8) nur noch 1,2 % Titan meßbar.

Die am Meßpunkt 7 ermittelten Restelementkonzentrationen liegen auch in diesem Fall niedriger als bei den α-Al$_2$O$_3$-gestrahlten Oberflächen. Wie bei sämtlichen anderen Titan-Keramik-Haftverbunden ist die Integrität der Kontaktzone als gut zu bezeichnen und die Metalloberfläche vollständig mit dem Bonder benetzt.

Tabelle 19:

| Ergebnisse der EDAX-Analyse für die Kombination Titan nicht α-Al$_2$O$_3$-gestrahlt/VITA TITANKERAMIK, TC | | | | | | | |
|---|---|---|---|---|---|---|---|
| | 2 µm im Metall (Meßpunkt 6) | | Kontaktzone (Meßpunkt 7) | | 2 µm in der Keramik (Meßpunkt 8) | | 5 µm in der Keramik (Meßpunkt 9) |
| | + ION | - ION | + ION | - ION | + ION | - ION | + ION | - ION |
| Ti | 98,1 | 100,0 | 79,1 | 96,5 | 1,2 | 12,1 | 1,0 | 1,3 |
| Si | 0,0 | 0,0 | 9,9 | 1,2 | 30,5 | 22,2 | 29,2 | 28,3 |
| Al | 0,0 | 0,0 | 1,2 | 0,0 | 2,8 | 2,3 | 2,7 | 5,1 |
| Rest | 1,9 | 0,0 | 6,8 | 1,9 | 55,1 | 45,3 | 57,3 | 52,1 |

Zusammenfassung

Die vorstehend beschriebenen Versuchsergebnisse zeigen, daß es durch das Verfahren zur Herstellung eines Titan-Keramik-Haftverbundes für Zahnersatz und bei dem daraus hergestellten Titan-Keramik-Haftverbund für Zahnersatz möglich ist, den Haftfestigkeitsverlust insbesondere bei zyklischen Temperaturlastwechseln weitgehend zu vermeiden.

Wie vorstehend dargelegt, wurden bei den Versuchen zwei verschiedene Titan-Keramikmassen (TIBOND und VITA TITANKERAMIK) auf gezogenes und gefrästes Titan Ti 2 aufgebrannt, wobei die Verblendfläche durch Implantation von Siliziumionen konditioniert wurde. Zusätzlich wurde dabei durch ein α-Al$_2$O$_3$ Strahl konventionell vorbereitetes Titan mit nicht α-Al$_2$O$_3$-gestrahltem Titan verglichen. Zu Vergleichszwecken dienten Prüfserien, deren Verblendfläche nicht durch Ionenstrahl konditioniert wurde. Nach 24 Stunden Trockenlagerung sowie nach künstlicher Alterung durch 5000 Temperaturlastwechselzyklen wurde die Haftfestigkeit der keramischen Verblendung im Schertest nach SCHMITZ-SCHULMEYER geprüft.

Ohne Ionenimplantation betrugen die Haftfestigkeitsverluste infolge Thermocycling statistisch signifikante 6,2 bis 17,3 %. Im Gegensatz dazu veränderten sich die Werte nach Thermocycling bei einer Ionen implantierten Titanoberfläche mit einer Ausnahme nicht statistisch signifikant (zwischen 1,7 % Haftfestigkeitsverlust und 0,8 % Haftfestigkeitserhöhung). Die bemerkenswerte Erhöhung besteht bei der VITA TITANKERAMIK auf nicht mit Korund gestrahltem Titan mit Ionenimplantation. Für diese Serie wurde eine statistisch hochsignifikante Zunahme der Haftfestigkeit von 39 % gemessen.

Bei Trockenlagerung wird die Haftfestigkeit der Keramik auf dem Titan durch die Ionenimplantation nur bei TIBOND statistisch signifikant verbessert, während sich bei VITA TITAN-KERAMIK keine Veränderung bzw. bei α-$Al_2O_3$-gestrahltem Titan eine geringfügige Verschlechterung feststellen läßt.

Nach Thermocycling liegen sämtliche Ionen modifizierten Prüfserien in ihren Werten statistisch signifikant über den nicht modifizierten Prüfserien.

Der Vergleich der α-$Al_2O_3$-gestrahlten Prüfkörper mit den nicht gestrahlten Prüfkörpern nach Thermocycling zeigt, daß die nicht gestrahlten Prüfkörper durch die Ionenimplantation Haftfestigkeitswerte in vergleichbarer Höhe der α-$Al_2O_3$-gestrahlten Prüfkörper ohne Ionenimplantation erreichen.

Die Ergebnisse der EDAX-Analyse und rasterelektronenmikroskopischen Untersuchungen der Titan-Keramik-Kontaktzone bestätigen die guten Resultate des Schertests nach SCHMITZ-SCHULMEYER. Die gegenläufige Titan-Siliziumdiffusion und die diesem Diffusionsverhalten folgende Ausbildung von schuppig-kristallinen Titansiliziden in der Keramik nahe der Titanoberfläche, die bei den nicht durch Ionenimplantation konditionierten Prüfserien feststellbar war, konnte durch die Siliziumimplantation vollständig blockiert werden.

Da durch die Ionenimplantation die Ausbildung der Titan-Silizium-Schicht 2 in der Oberfläche 1 des Körpers aus Reintitan durchgeführt wird, kann der Grundkörper für einen Zahnersatz vor der Implantation vollständig maßhaltig gestaltet werden. Anschließend erfolgt dann lediglich noch das Aufbrennen der Dentalkeramik.

Dabei kann die Titan-Silizium-Schicht 2 auf den gesamten Körper aufgebracht werden. Das Aufbrennen der Dentalkeramik bei Zahnersatz erfolgt auf einzelnen Abschnitten, insbesondere im Zahnbereich sowie im Bereich des Schleimhautkontaktes. Durch die aufgebrachte Titan-Silizium-Schicht 2 wird gleichzeitig der Austritt von Titanionen aus dem Grundkörper des Zahnersatzes geblockt.

Das Titan-Keramik-Haftverbundsystem ist auch für Körper bzw. Werkstücke, die in einem Hochtemperaturbereich von 600 bis 3600 °C eingesetzt werden, vorteilhaft verwendbar.

Hierbei werden ebenfalls in die Oberfläche 1 eines Körpers aus Reintitan oder einer Titanlegierung durch eine Ionenimplantation mit Ionenstrahlen 3 zwischen die Atome 5 des Titans oder der Atome 5 der Titanlegierung Siliziumionen 4 eingebracht. Es wird in der Oberfläche 1 des Körpers in der Durchdringungsschicht der Ionenimplantation eine Titan-Silizium-Schicht 2 ausgebildet. Auf die Titan-Silizium-Schicht 2 werden kristalline, nichtmetallisch-anorganische Werkstoffe aufgetragen, wobei mit den Werkstoffen ein Haftverbundsystem hergestellt wird. Die Siliziumionen 4 können dabei in Form von Siliziumaggregaten in die Titan-Silizium-Schicht 2 eingelagert sein.

Die Ionenimplantation von Siliziumionen 4 in die Oberfläche 1 des Körpers aus Reintitan oder einer Titanlegierung erfolgt mit einer Ionendosis von 1 x $10^8$ bis 1 x $10^{18}$ Atomen/cm$^2$, vorzugsweise 9 x $10^{16}$ Atomen/cm$^2$ und einer Ionenenergie von 30 bis 400 KeV, vorzugsweise 150 KeV.

Die kristallinen, nichtmetallisch-anorganischen Werkstoffe bestehen aus glaskeramischen Werkstoffen, nichtoxidischkeramischen Werkstoffen oder oxidisch-keramischen Werkstoffen. Hierdurch wird erreicht, daß die spezifischen Werkstoffeigenschaften des Titans, nämlich seine hohe Festigkeit bei einem geringen Gewicht, auch für Werkstücke genutzt werden können, die in einem Hochtemperaturbereich einzusetzen sind. Ein bevorzugtes Einsatzgebiet ist hierbei die Fertigung von Werkstücken für Motoren und Triebwerke im Kraftfahrzeugbau sowie in der Luft- und Raumfahrt.

Besonders vorteilhaft ist auch für dieses Einsatzgebiet, daß der Körper aus Reintitan oder einer Titanlegierung vor der Ionenimplantation als ein Werkstück zur Verwendung in einem Hochtemperaturbereich von 600 bis 3600 °C ausgebildet ist und außer dem thermischen Auftragen eines kristallinen, nichtmetallisch-anorganischen Werkstoffes keine weitere Behandlung des Körpers nach der Ausbildung der Titan-Silizium-Schicht 2 in der Oberfläche 1 des Körpers erfolgt.

Durch das erfindungsgemäße Titan-Keramik-Haftverbundsystem wird auch im Hochtemperaturbereich die Haftfestigkeit bei wechselnden thermischen Belastungen verbessert.

Der Einsatz des Titan-Keramik-Haftverbundsystems ist dabei nicht auf den angegebenen Bereich beschränkt, sondern erstreckt sich auf alle Gebiete, bei denen ein fester Verbund zwischen den aufzubringenden nichtmetallisch-anorganischen Werkstoffen und dem Grundkörper aus Titan auch bei thermischen Belastungen erreicht werden soll. Hiervon ausgehend ist ein weiteres Einsatzgebiet der chemische Apparatebau, da auch hier teilweise chemische Prozesse in einem hohen Temperaturbereich ablaufen.

Der aufgetragene Werkstoff verhindert dabei insbesondere auch unerwünschte chemische Reaktionen mit dem Material des Gefäßes, in dem die Prozesse ablaufen.

**Patentansprüche**

**1.** Verfahren zur Herstellung eines Titan-Keramik-Haftverbundsystems, **dadurch gekennzeichnet, daß** in die Oberfläche (1) eines Körpers aus Reintitan oder einer Titanlegierung durch eine Ionenimplantation mit Ionenstrahlen (3) zwischen die Atome (5) des Titans oder die Atome (5) der Titanlegierung Siliziumionen (4) eingebracht werden, durch die in der Oberfläche (1) des Körpers in der Durchdringungsschicht der Ionenimplantation eine Titan-Silizium-Schicht (2) ausgebildet wird, auf die kristalline, nichtmetallisch-anorganische Werkstoffe thermisch aufgetragen werden und mit den Werkstoffen ein Haftverbund hergestellt wird.

2. Verfahren nach Anspruch 1, **dadurch gekennzeichnet, daß** die Siliziumionen (4) in Form von Siliziumaggregaten in die Titan-Silizium-Schicht (2) eingelagert werden.

3. Verfahren nach Anspruch 1, **dadurch gekennzeichnet, daß** die kristallinen, nichtmetallisch-anorganischen Werkstoffe aus glaskeramischen Werkstoffen, nichtoxidischen keramischen Werkstoffen oder oxidischen keramischen Werkstoffen bestehen.

4. Verfahren nach Anspruch 1, **dadurch gekennzeichnet, daß** als Titanlegierung eine Titan-Vanadium-Aluminium-Legierung folgender Zusammensetzung eingesetzt wird:
Ti-6Al-4V

5. Verfahren nach Anspruch 1, **dadurch gekennzeichnet, daß** als Titanlegierung eine den speziellen Anforderungen des Einsatzgebietes und der möglichen Verfahrenstechnik entsprechenden Titanlegierung verwendet werden.

6. Verfahren nach Anspruch 1, **dadurch gekennzeichnet, daß** die Ionenimplantation von Siliziumionen (4) in die Oberfläche (1) des Körpers aus Reintitan oder einer Titanlegierung mit einer Ionendosis von $1 \times 10^8$ bis $1 \times 10^{18}$ Atomen/cm$^2$ und einer Ionenenergie von 30 bis 400 KeV durchgeführt wird.

7. Verfahren nach Anspruch 6, **dadurch gekennzeichnet, daß** die Ionenimplantation von Siliziumionen (4) in die Oberfläche (1) des Körpers aus Reintitan oder einer Titanlegierung mit einer Ionendosis von $9 \times 10^{16}$ Atomen/cm$^2$ und einer Ionenenergie von 150 KeV durchgeführt wird.

8. Verfahren zur Herstellung eines Titan-Keramik-Haftverbundsystems nach Anspruch 1, **dadurch gekennzeichnet, daß** zur Verwendung für einen Zahnersatz in die Oberfläche (1) eines Körpers aus Reintitan durch eine Ionenimplantation mit Ionenstrahlen (3) zwischen die Atome (5) des Titans Siliziumionen (4) eingebracht werden, durch die in der Oberfläche (1) des Körpers in der Durchdringungsschicht der Ionenimplantation eine Titan-Silizium-Schicht (2) ausgebildet wird, auf die eine für die Verblendung von Titan bestimmte Dentalkeramik aufgebrannt wird.

9. Verfahren nach Anspruch 8, **dadurch gekennzeichnet, daß** die Ionenimplantation von Siliziumionen (4) in die Oberfläche (1) des Körpers aus Reintitan mit einer Ionendosis von $1 \times 10^{12}$ bis $1 \times 10^{18}$ Atomen/cm$^2$ und einer Ionenenergie von 30 bis 400 KeV durchgeführt wird.

10. Verfahren nach Anspruch 9, **dadurch gekennzeichnet, daß** die Ionenimplantation von Siliziumionen (4) in die Oberfläche (1) des Körpers aus Reintitan mit einer Ionendosis von $3 \times 10^{17}$ Atomen/cm$^2$ und einer Ionenenergie von 150 KeV durchgeführt wird.

11. Verfahren nach Anspruch 8 bis 10, **dadurch gekennzeichnet, daß** das Reintitan in folgende Anteile (Angaben in Masse-%) enthält:

| | |
|---|---|
| $O_{max}$ | 0,12 |
| $N_{max}$ | 0,05 |
| $C_{max}$ | 0,06 |
| $H_{max}$ | 0,013 |
| Ti | Rest |

12. Verfahren nach Anspruch 8 bis 11, **dadurch gekennzeichnet, daß** vor der Ionenimplantation der Oberfläche (1) des Körpers aus Reintitan die oberflächige Titanoxidschicht durch eine spanabhebende Bearbeitung entfernt wird und anschließend durch gemahlenes, monokristallines Silizium ($Si_{mon}$) mit einer Maschgröße von 50 bis 300 μm unter einer Schutzgasatmosphäre aufgerauht wird.

13. Verfahren nach Anspruch 8 bis 10, **dadurch gekennzeichnet, daß** nach der Entfernung der Titanoxidschicht von der Oberfläche (1) des Körpers aus Reintitan die Oberfläche (1) durch Abstrahlen mit Korund ($\alpha$-Al$_2$O$_3$) der Korngröße 50 - 250 μm aufgerauht wird.

14. Verfahren nach einem oder mehreren der Ansprüche 8 bis 13, **dadurch gekennzeichnet, daß** der Körper aus Reintitan vor der Ionenimplantation vollständig als ein Grundkörper für einen Zahnersatz gestaltet wird und außer dem Aufbrennen der Dentalkeramik keine weitere Bearbeitung des Körpers nach der Ausbildung der Titan-Silizium-Schicht (2) in der Oberfläche (1) des Körpers durchgeführt wird.

15. Verfahren nach einem oder mehreren der Ansprüche 8 bis 14, **dadurch gekennzeichnet, daß** in der gesamten Oberfläche (1) des als Grundkörper für einen Zahnersatz gestalteten Körpers aus Reintitan die Titan-Silizium-Schicht (2) ausgebildet wird und die für die Verblendung von Titan bestimmte Dentalkeramik auf einzelne Abschnitte der Oberfläche (1) aufgebrannt wird, wobei die Dentalkeramik mindestens auf die, die Zahnbereiche und die Bereiche des Schleimhautkontaktes bildenden, Abschnitte des Grundkörpers für einen Zahnersatz aufgebrannt wird.

16. Verfahren nach einem oder mehreren der Ansprüche 8 bis 15, **dadurch gekennzeichnet, daß** die für die Verblendung von Titan bestimmte Dentalkeramik in vier Brennzyklen

    1. Zyklus: Bonder und/oder Washbrand-Masse
    2. Zyklus: Grundmassebrand
    3. Zyklus: Dentinbrand
    4. Zyklus: Glanzbrand

auf die Titan-Silizium-Schicht (2) aufgebrannt wird.

17. Verfahren zur Herstellung eines Titan-Keramik-Haftverbundsystems nach Anspruch 1, **dadurch gekennzeichnet, daß** zur Verwendung in einem Hochtemperaturbereich von 600 bis 3600 °C in die Oberfläche (1) eines Körpers aus Reintitan oder einer Titanlegierung durch eine Ionenimplantation mit Ionenstrahlen (3) zwischen die Atome (5) des Titans oder die Atome (5) der Titanlegierung Siliziumionen (4) eingebracht werden, durch die in der Oberfläche (1) des Körpers in der Durchdringungsschicht der Ionenimplantation eine Titan-Silizium-Schicht (2) ausgebildet wird, auf die kristalline, nichtmetallisch-anorganische Werkstoffe thermisch aufgetragen werden und mit den Werkstoffen ein Haftverbund hergestellt wird.

18. Verfahren nach Anspruch 17, **dadurch gekennzeichnet, daß** die Siliziumionen (4) in Form von Siliziumaggregaten in die Titan-Siliziumschicht (2) eingelagert werden.

19. Verfahren nach Anspruch 17, **dadurch gekennzeichnet, daß** die kristallinen, nichtmetallisch-anorganischen Werkstoffe aus glaskeramischen Werkstoffen, nichtoxidischen keramischen Werkstoffen oder oxidischen keramischen Werkstoffen bestehen.

20. Verfahren nach Anspruch 17, **dadurch gekennzeichnet, daß** die Ionenimplantation von Siliziumionen (4) in die Oberfläche (1) des Körpers aus Reintitan oder einer Titanlegierung mit einer Ionendosis von 1 x 10$^8$ bis 1 x 10$^{16}$ Atomen/cm$^2$ und einer Ionenenergie von 30 bis 400 KeV durchgeführt wird.

21. Verfahren nach Anspruch 20, **dadurch gekennzeichnet, daß** die Ionenimplantation von Siliziumionen (4) in die Oberfläche (1) des Körpers aus Reintitan oder einer Titanlegierung mit einer Ionendosis von 9 x 10$^{16}$ Atomen/cm$^2$ und einer Ionenenergie von 150 KeV durchgeführt wird.

22. Verfahren nach Anspruch 17 bis 21, **dadurch gekennzeichnet, daß** der Körper aus Reintitan oder einer Titanlegierung vor der Ionenimplantation als ein Werkstück zur Verwendung in einem Hochtemperaturbereich von 600 bis 3600°C ausgebildet wird und außer dem thermischen Auftragen eines kristallinen, nichtmetallisch-anorganischen Werkstoffes keine weitere Bearbeitung des Körpers nach der Ausbildung der Titan-Silizium-Schicht (2) in der Oberfläche (1) des Körpers durchgeführt wird.

23. Verfahren nach Anspruch 22, **dadurch gekennzeichnet, daß** das Werkstück in Motoren und Triebwerken des Kraftfahrzeugbaus sowie der Luft- und Raumfahrt eingesetzt wird.

24. Titan-Keramik-Haftverbundsystem, hergestellt nach dem Verfahren nach Anspruch 1, **dadurch gekennzeichnet,**

**EP 0 910 684 B1**

**daß** die Oberfläche (1) eines Körpers aus Reintitan oder einer Titanlegierung als eine Titan-Silizium-Schicht (2) ausgebildet ist, wobei die Siliziumionen (4) durch eine Ionenimplantation zwischen die Atome (5) des Titans oder die Atome (5) der Titanlegierung eingebracht sind und wobei auf die Titan-Silizium-Schicht (2) ein kristalliner, nichtmetallisch-anorganischer Werkstoff thermisch aufgetragen ist.

25. Titan-Keramik-Haftverbundsystem nach Anspruch 24, **dadurch gekennzeichnet, daß** die Siliziumionen (4) zur Ausbildung der Titan-Silizium-Schicht (2) in der Oberfläche (1) des Körpers aus Reintitan oder einer Titanlegierung mit einer Ionendosis von 1 x 10$^8$ bis 1 x 10$^{18}$ Atomen/cm$^2$ und einer Ionenenergie von 30 bis 400 KeV eingebracht sind.

26. Titan-Keramik-Haftverbundsystem nach Anspruch 25, **dadurch gekennzeichnet, daß** die Siliziumionen (4) in der Oberfläche (1) des Körpers aus Reintitan oder einer Titanlegierung mit einer Ionendosis von 9 x 10$^{16}$ Atomen/cm$^2$ und einer Ionenenergie von 150 KeV eingebracht sind.

27. Titan-Keramik-Haftverbundsystem, hergestellt nach dem Verfahren nach Anspruch 8, **dadurch gekennzeichnet, daß** zur Verwendung für einen Zahnersatz die Oberfläche (1) eines Körpers aus Reintitan als eine Titan-Silizium-Schicht (2) ausgebildet ist, wobei Siliziumionen (4) durch eine Ionenimplantation zwischen die Titanatome (5) des Reintitans eingebracht sind und wobei auf die Titan-Silizium-Schicht (2) eine für die Verblendung von Titan bestimmte Dentalkeramik aufgebrannt ist.

28. Titan-Keramik-Haftverbundsystem nach Anspruch 27, **dadurch gekennzeichnet, daß** die Siliziumionen (4) zur Ausbildung der Titan-Silizium-Schicht (2) in der Oberfläche (1) des Körpers aus Reintitan mit einer Ionendosis von 1 x 10$^{12}$ bis 1 x 10$^{18}$ Atomen/cm$^2$ an Siliziumionen (4) und einer Ionenenergie von 30 bis 400 KeV eingebracht sind.

29. Titan-Keramik-Haftverbundsystem nach Anspruch 28, **dadurch gekennzeichnet, daß** die Siliziumionen (4) zur Ausbildung der Titan-Silizium-Schicht (2) in der Oberfläche (1) des Körpers aus Reintitan mit einer Ionendosis von 3 x 10$^{17}$ Atomen/cm$^2$ und einer Ionenenergie von 150 KeV eingebracht sind.

30. Titan-Keramik-Haftverbundsystem nach den Ansprüchen 27 bis 29, **dadurch gekennzeichnet, daß** das Reintitan folgende Zusammensetzung (Angaben in Masse-%) aufweist:

| | |
|---|---|
| $O_{max}$ | 0, 12 |
| $N_{max}$ | 0,05 |
| $C_{max}$ | 0,06 |
| $H_{max}$ | 0,013 |
| Ti | Rest |

31. Titan-Keramik-Haftverbundsystem nach einem oder mehreren der Ansprüche 27 bis 30, **dadurch gekennzeichnet, daß** der Körper aus Reintitan vor der Ausbildung seiner Oberfläche (1) als eine Titan-Silizium-Schicht (2) vollständig als ein Grundkörper für einen Zahnersatz gestaltet ist, auf dem nach der Ausbildung der Titan-Silizium-Schicht (2) als einzige Bearbeitung die Dentalkeramik durch Aufbrennen aufgebracht ist.

32. Titan-Keramik-Haftverbundsystem nach einem oder mehreren der Ansprüche 27 bis 31, **dadurch gekennzeichnet, daß** in der gesamten Oberfläche (1) des als Grundkörper für einen Zahnersatz gestalteten Körpers aus Reintitan die Titan-Silizium-Schicht (2) ausgebildet ist und die für die Verblendung von Titan bestimmte Dentalkeramik auf einzelne Abschnitte der Oberfläche (1) aufgebrannt ist, wobei die Dentalkeramik mindestens auf die, die Zahnbereiche und die Bereiche des Schleimhautkontaktes bildenden, Abschnitte des Grundkörpers für einen Zahnersatz aufgebrannt ist.

33. Titan-Keramik-Haftverbundsystem, hergestellt nach dem Verfahren nach Anspruch 17, **dadurch gekennzeichnet, daß** zur Verwendung in einem Hochtemperaturbereich von 600 bis 3600 °C die Oberfläche (1) eines Körpers aus Reintitan oder einer Titanlegierung als eine Titan-Silizium-Schicht (2) ausgebildet ist, wobei die Siliziumionen (4) durch eine Ionenimplantation zwischen die Atome (5) des Titans oder die Atome (5) der Titanlegierung eingebracht sind und wobei auf die Titan-Silizium-Schicht (2) ein kristalliner, nichtmetallisch-anorganischer Werkstoff thermisch aufgetragen ist.

34. Titan-Keramik-Haftverbundsystem nach Anspruch 33, **dadurch gekennzeichnet, daß** die Siliziumionen (4) in

25

Form von Siliziumaggregaten in die Titan-Silizium-Schicht (2) eingelagert sind.

**35.** Titan-Keramik-Haftverbundsystem nach Anspruch 33, **dadurch gekennzeichnet, daß** die kristallinen, nichtmetallisch-anorganischen Werkstoffe aus glaskeramischen Werkstoffen, nichtoxidischen keramischen Werkstoffen oder oxidischen keramischen Werkstoffen bestehen.

**36.** Titan-Keramik-Haftverbundsystem nach Anspruch 33, **dadurch gekennzeichnet, daß** die Ionenimplantation von Siliziumionen (4) in die Oberfläche (1) des Körpers aus Reintitan oder einer Titanlegierung mit einer Ionendosis von 1 x $10^8$ bis 1 x $10^{18}$ Atomen/cm$^2$ und einer Ionenenergie von 30 bis 400 KeV durchgeführt wird.

**37.** Titan-Keramik-Haftverbundsystem nach Anspruch 36, **dadurch gekennzeichnet, daß** die Ionenimplantation von Siliziumionen (4) in die Oberfläche (1) des Körpers aus Reintitan oder einer Titanlegierung mit einer Ionendosis von 9 x $10^{16}$ Atomen/cm$^2$ und einer Ionenenergie von 150 KeV durchgeführt wird.

**38.** Titan-Keramik-Haftverbundsystem nach Anspruch 33 bis 37, **dadurch gekennzeichnet, daß** der Körper aus Reintitan oder einer Titanlegierung vor der Ionenimplantation als ein Werkstück zur Verwendung in einem Hochtemperaturbereich von 600 bis 3600°C ausgebildet ist und außer dem thermischen Auftragen eines kristallinen, nichtmetallisch-anorganischen Werkstoffes keine weitere Bearbeitung des Körpers nach der Ausbildung der Titan-Silizium-Schicht (2) in der Oberfläche (1) des Körpers erfolgt.

**39.** Titan-Keramik-Haftverbundsystem nach Anspruch 38, **dadurch gekennzeichnet, daß** das Werkstück in Motoren und Triebwerken des Kraftfahrzeugbaus sowie der Luft- und Raumfahrt einsetzbar ist.

**Claims**

**1.** A process for producing a titanium-ceramic adhesive composite system **characterised in that** silicon ions are introduced into the surface (1) of a pure titanium or titanium-alloy structure by ion implantation with ion beams (3) between the atoms (5) of the titanium or the atoms (5) of the titanium alloy, by means of which a titanium-silicon layer (2) is formed in the surface (1) of the structure in the penetration layer of ion implantation, and crystalline non-metallic inorganic materials are thermally applied on to the titanium-silicon layer and an adhesive bond is made with the materials.

**2.** A process according to claim 1, **characterised in that** the silicon ions (4) are incorporated in the form of silicon aggregates in the titanium-silicon layer (2).

**3.** A process according to claim 1, **characterised in that** the crystalline non-metallic inorganic materials consist of glass-ceramic materials, non-oxidic ceramic materials or oxidic ceramic materials.

**4.** A process according to claim 1, **characterised in that** the titanium alloy used is a titanium-vanadium-aluminium alloy having the following composition:
Ti-6A1-4V

**5.** A process according to claim 1, **characterised in that** the titanium alloy is a titanium alloy conforming to the special requirements of the application and the possible production technique.

**6.** A process according to claim 1, **characterised in that** the implantation of silicon ions (4) into the surface (1) of the pure titanium or titanium-alloy structure is performed at an ion dosage of 1 x $10^8$ to 1 x $10^{18}$ atoms/cm$^2$ and an ion energy of 30 to 400 KeV.

**7.** A process according to claim 6, **characterised in that** the implantation of silicon ions (4) into the surface (1) of the pure titanium or titanium-alloy structure is performed at an ion dosage of 9 x $10^{16}$ atoms/cm$^2$ and an ion energy of 150 KeV.

**8.** A process of producing a titanium-ceramic adhesive composite system according to claim 1, **characterised in that** for use in a dental prosthesis, silicon ions (4) are introduced into the surface (1) of a pure titanium structure by ion implantation with ion beams (3) between the atoms (5) of titanium, as a result of which a titanium-silicon layer (2) is formed in the surface (1) of the structure in the penetration layer of the ion implantation, and a dental ceramic for titanium veneering is fired on the titanium-silicon layer.

9. A process according to claim 8, **characterised in that** the implantation of silicon ions (4) into the surface (1) of the pure titanium structure is performed at an ion dosage of $1 \times 10^{12}$ to $1 \times 10^{18}$ atoms/cm$^2$ and an ion energy of 30 to 400 KeV.

10. A process according to claim 9, **characterised in that** the implantation of silicon ions (4) into the surface (1) of the pure titanium structure is carried out at an ion dosage of $3 \times 10^{17}$ atoms/cm$^2$ and an ion energy of 150 KeV.

11. A process according to claims 8 to 10, **characterised in that** the pure titanium contains the following proportions (in % by mass): $O_{max}$ 0.12

| | |
|---|---|
| $N_{max}$ | 0.05 |
| $C_{max}$ | 0.06 |
| $H_{max}$ | 0.013 |
| Ti | Remainder |

12. A process according to claims 8 to 11, **characterised in that** before the ion implantation of the surface (1) of the pure titanium structure, the surface titanium oxide layer is removed by machining and is subsequently roughened in a protective gas atmosphere by ground monocrystalline silicon ($Si_{mon}$) having a mesh size of 50 to 300 µm.

13. A process according to claims 8 to 10, **characterised in that** after removal of the titanium oxide layer from the surface (1) of the pure titanium structure, the surface (1) is roughened by blasting with corundum ($\alpha$-$Al_2O_3$) having a particle size of 50 - 250 µm.

14. A process according to one or more of claims 8 to 13, **characterised in that** the pure titanium structure, before the ion implantation, is completely formed as a base member for a dental prosthesis and, apart from firing on the dental ceramic, no further treatment of the structure is carried out after the titanium-silicon layer (2) has formed in the surface (1) of the structure.

15. A process according to one or more of claims 8 to 14, **characterised in that** the titanium-silicon layer (2) is formed in the entire surface (1) of the pure titanium structure formed as the base member for a dental prosthesis, and the dental ceramic for the titanium veneering is fired on individual portions of the surface (1), the dental ceramic being fired on at least those portions of the base member for a dental prosthesis which form the tooth regions and the regions of contact with the mucous membrane.

16. A process according to one or more of claims 8 to 15, **characterised in that** the dental ceramic for the titanium veneering is fired on the titanium-silicon layer (2) in four firing cycles:

    1st cycle: bonder and/or wash-firing material;
    2nd cycle: base material firing;
    3rd cycle: dentine firing;
    4th cycle: gloss firing.

17. A process for producing a titanium-ceramic adhesive composite system according to claim 1, **characterised in that**, for use in a high temperature range from 600 to 3600°C, silicon ions (4) are introduced into the surface (1) of a pure titanium or titanium-alloy structure by ion implantation with ion beams (3) between the atoms (5) of the titanium or the atoms (5) of the titanium alloy, as a result of which a titanium-silicon layer (2) is formed in the surface (1) of the structure in the penetration layer of the ion implantation, and crystalline non-metallic inorganic materials are thermally applied to the titanium-silicon layer and an adhesive bond is made with the materials.

18. A process according to claim 17, **characterised in that** the silicon ions (4) are incorporated in the form of silicon aggregates in the titanium-silicon layer (2).

19. A process according to claim 17, **characterised in that** the crystalline non-metallic inorganic materials consist of glass-ceramic materials, non-oxidic ceramic materials or oxidic ceramic materials.

**20.** A process according to claim 17, **characterised in that** the implantation of silicon ions (4) into the surface (1) of the pure titanium or titanium-alloy structure is performed at an ion dosage of $1 \times 10^8$ to $1 \times 10^{18}$ atoms/cm$^2$ and an ion energy of 30 to 400 KeV.

**21.** A process according to claim 20, **characterised in that** the implantation of silicon ions (4) into the surface (1) of the pure titanium or titanium alloy structure is performed at an ion dosage of $9 \times 10^{16}$ atoms/cm$^2$ and an ion energy of 150 KeV.

**22.** A process according to claims 17 to 21, **characterised in that** before the ion implantation, the pure titanium or titanium-alloy structure is made in the form of a workpiece for use in a high-temperature range of 600 to 3600°C and, apart from thermal application of a crystalline non-metallic inorganic material, no further treatment of the structure is carried out after the titanium-silicon layer (2) has been formed in the surface (1) of the structure.

**23.** A process according to claim 22, **characterised in that** the workpiece is used in engines and power units of motor vehicles and in air and space travel.

**24.** A titanium-ceramic adhesive composite system produced by the process according to claim 1, **characterised in that** the surface (1) of a pure titanium or titanium-alloy structure is made in the form of a titanium-silicon layer (2), the silicon ions (4) being introduced by ion implantation between the atoms (5) of the titanium or the atoms (5) of the titanium alloy and wherein a crystalline non-metallic inorganic material is thermally deposited on to the titanium-silicon layer (2).

**25.** A titanium-ceramic adhesive composite system according to claim 24, **characterised in that** the silicon ions (4) for producing the titanium-silicon layer (2) are introduced into the surface (1) of the pure titanium or titanium-alloy structure at an ion dosage of $1 \times 10^8$ to $1 \times 10^{18}$ atoms/cm$^2$ and an ion energy of 30 to 400 KeV.

**26.** A titanium-ceramic adhesive composite system according to claim 25, **characterised in that** the silicon ions (4) are introduced into the surface (1) of the pure titanium or titanium-alloy structure at an ion dosage of $9 \times 10^{16}$ atoms/cm$^2$ and an ion energy of 150 KeV.

**27.** A titanium-ceramic adhesive composite system produced by the process according to claim 8, **characterised in that** for use as a dental prosthesis, the surface (1) of a pure titanium structure is formed as a titanium-silicon layer (2), wherein silicon ions (4) are introduced by ion implantation between the atoms (5) of the pure titanium and wherein a dental ceramic intended for titanium veneering is fired on the titanium-silicon layer (2).

**28.** A titanium-ceramic adhesive composite system according to claim 27, **characterised in that** the silicon ions (4) for forming the titanium-silicon layer (2) are introduced into the surface (1) of the pure titanium structure at an ion dosage of $1 \times 10^{12}$ to $1 \times 10^{18}$ atoms/cm$^2$ of silicon ions (4) and an ion energy of 30 to 400 KeV.

**29.** A titanium-ceramic adhesive composite system according to claim 28, **characterised in that** the silicon ions (4) for forming the titanium-silicon layer (2) are introduced into the surface (1) of the pure titanium structure at an ion dosage of $3 \times 10^{17}$ atoms/cm$^2$ and an ion energy of 150 KeV.

**30.** A titanium-ceramic adhesive composite system according to claims 27 to 29, **characterised in that** the pure titanium has the following composition (as % by mass):

| | |
|---|---|
| $O_{max}$ | 0.12 |
| $N_{max}$ | 0.05 |
| $C_{max}$ | 0.06 |
| $H_{max}$ | 0.013 |
| Ti | Remainder |

**31.** A titanium-ceramic adhesive composite system according to one or more of claims 27 to 30, **characterised in that** the pure titanium structure, before its surface (1) has been converted into a titanium-silicon layer (2), is made completely in the form of a base member for a dental prosthesis, and after the titanium-silicon layer (2) has formed, the dental ceramic is applied thereto by firing in a single treatment operation.

**32.** A titanium-ceramic adhesive composite system according to one or more of claims 27 to 31, **characterised in that** the titanium-silicon layer (2) is formed in the entire surface (1) of the pure titanium structure, which is in the form of the base member for a dental prosthesis, and the dental ceramic intended for the titanium veneering is fired on individual portions of the surface (1), the dental ceramic being fired at least on those portions of the base member for a dental prosthesis which form the tooth regions and the regions of contact with the mucous membrane.

**33.** A titanium-ceramic adhesive composite system produced by the process according to claim 17, **characterised in that**, for use in a high-temperature range from 600 to 3600°C, the surface (1) of a pure titanium or titanium-alloy structure is formed as a titanium-silicon layer (2), the silicon ions (4) being introduced by ion implantation between the atoms (5) of the titanium or the atoms (5) of the titanium alloy and wherein a crystalline non-metallic inorganic material is thermally deposited on to the titanium-silicon layer (2).

**34.** A titanium-ceramic adhesive composite system according to claim 33, **characterised in that** the silicon ions (4) are incorporated in the form of silicon aggregates into the titanium-silicon layer (2).

**35.** A titanium-ceramic adhesive composite system according to claim 33, **characterised in that** the crystalline non-metallic inorganic materials consist of glass-ceramic materials, non-oxidic ceramic materials or oxidic ceramic materials.

**36.** A titanium-ceramic adhesive composite system according to claim 33, **characterised in that** the implantation of silicon ions (4) into the surface (1) of the pure titanium or titanium-alloy structure is carried out at an ion dosage of $1 \times 10^8$ to $1 \times 10^{18}$ atoms/cm$^2$ and an ion energy of 30 to 400 KeV.

**37.** A titanium-ceramic adhesive composite system according to claim 36, **characterised in that** the implantation of silicon ions (4) into the surface (1) of the pure titanium or titanium-alloy structure is carried out at an ion dosage of $9 \times 10^{16}$ atoms/cm$^2$ and an ion energy of 150 KeV.

**38.** A titanium-ceramic adhesive composite system according to claims 33 to 37, **characterised in that** before the ion implantation, the pure titanium or titanium-alloy structure is made in the form of a workpiece for use in a high-temperature range from 600 to 3600°C and, apart from thermal application of a crystalline, non-metallic inorganic material, no further treatment of the structure is carried out after the titanium-silicon layer (2) has formed in the surface (1) of the structure.

**39.** A titanium-ceramic adhesive composite system according to claim 38, **characterised in that** the workpiece is usable in engines and power units in motor-vehicle construction and in air and space travel.

**Revendications**

**1.** Procédé de fabrication d'un système composite adhésif titane-céramique, **caractérisé par le fait que** dans la surface (1) d'un corps en titane pur ou en alliage de titane sont introduits par une implantation ionique avec des faisceaux ioniques (3) entre les atomes (5) du titane ou les atomes (5) de l'alliage de titane des ions silicium (4) qui forment dans la surface (1) du corps dans la couche de pénétration de l'implantation ionique une couche de titane-silicium (2) sur laquelle sont appliqués par voie thermique des matériaux cristallins inorganiques non métalliques et une liaison par adhérence est produite avec ces matériaux.

**2.** Procédé selon la revendication 1, **caractérisé par le fait que** les ions silicium (4) sont introduits dans la couche de titane-silicium (2) sous la forme d'agrégats de silicium.

**3.** Procédé selon la revendication 1, **caractérisé par le fait que** les matériaux cristallins inorganiques non métalliques sont constitués de matériaux vitrocéramiques, de matériaux céramiques non d'oxydes ou de matériaux céramiques d'oxydes.

**4.** Procédé selon la revendication 1, **caractérisé par le fait que** comme alliage de titane est employé un alliage titane-vanadium-aluminium ayant la composition suivante :
Ti-6A1-4V.

**5.** Procédé selon la revendication 1, **caractérisé par le fait que** comme alliage de titane est utilisé un alliage de titane répondant aux exigences spéciales du domaine d'emploi et correspondant à la technique possible de procédé.

**6.** Procédé selon la revendication 1, **caractérisé par le fait que** l'implantation ionique d'ions silicium (4) dans la surface (1) du corps en titane pur ou en alliage de titane est effectuée avec une dose ionique de 1 x 10$^8$ à 1 x 10$^{18}$ atomes/cm$^2$ et une énergie ionique de 30 à 400 keV.

**7.** Procédé selon la revendication 6, **caractérisé par le fait que** l'implantation ionique d'ions silicium (4) dans la surface (1) du corps en titane pur ou en alliage de titane est effectuée avec une dose ionique de 9 x 10$^{16}$ atomes/cm$^2$ et une énergie ionique de 150 keV.

**8.** Procédé de fabrication d'un système composite adhésif titane-céramique selon la revendication 1, **caractérisé par le fait que** pour l'utilisation pour une prothèse dentaire, dans la surface (1) d'un corps en titane pur sont introduits par une implantation ionique avec des faisceaux ioniques (3) entre les atomes (5) du titane des ions silicium (4) qui forment dans la surface (1) du corps dans la couche de pénétration de l'implantation ionique une couche de titane-silicium (2) sur laquelle est appliquée par cuisson une céramique dentaire destinée à recouvrir le titane.

**9.** Procédé selon la revendication 8, **caractérisé par le fait que** l'implantation ionique d'ions silicium (4) dans la surface (1) du corps en titane pur est effectuée avec une dose ionique de 1 x 10$^{12}$ à 1 x 10$^{18}$ atomes/cm2 et une énergie ionique de 30 à 400 keV.

**10.** Procédé selon la revendication 9, **caractérisé par le fait que** l'implantation ionique d'ions silicium (4) dans la surface (1) du corps en titane pur est effectuée avec une dose ionique de 3 x 10$^{17}$ atomes/cm$^2$ et une énergie ionique de 150 keV.

**11.** Procédé selon les revendications 8 à 10, **caractérisé par le fait que** le titane pur contient dans les proportions suivantes (valeurs en % en masse) :

| | |
|---|---|
| $O_{max}$ | 0,12 |
| $N_{max}$ | 0,05 |
| $C_{max}$ | 0,06 |
| $H_{max}$ | 0,013 |
| Ti | le reste |

**12.** Procédé selon les revendications 8 à 11, **caractérisé par le fait qu'**avant l'implantation ionique de la surface (1) du corps en titane pur, la couche superficielle d'oxyde de titane est enlevée par usinage par enlèvement de matière et la nouvelle surface est ensuite rendue rugueuse par du silicium monocristallin broyé (Simon) ayant une grandeur de maille de 50 à 300 microns sous une atmosphère de gaz protecteur.

**13.** Procédé selon les revendications 8 à 10, **caractérisé par le fait qu'**après l'enlèvement de la couche d'oxyde de titane de la surface (1) du corps en titane pur, la surface (1) est rendue rugueuse par projection de corindon ($Al_2O_3$) de grosseur de grain 50 - 250 microns.

**14.** Procédé selon une ou plusieurs des revendications 8 à 13, **caractérisé par le fait que** le corps en titane pur est, avant l'implantation ionique, réalisé entièrement sous la forme d'un corps de base pour une prothèse dentaire, et en dehors de l'application par cuisson de la céramique dentaire, il n'est pas effectué d'autre traitement du corps après la formation de la couche de titane-silicium (2) dans la surface (1) du corps.

**15.** Procédé selon une ou plusieurs des revendications 8 à 14, **caractérisé par le fait que** la couche de titane-silicium (2) est formée dans toute la surface (1) du corps en titane pur réalisé comme corps de base pour une prothèse dentaire, et la céramique dentaire destinée à recouvrir le titane est appliquée par cuisson sur certaines parties de la surface (1), la céramique dentaire étant appliquée par cuisson au moins sur les parties du corps de base pour une prothèse dentaire qui forment les zones des dents et les zones de contact avec les muqueuses.

**16.** Procédé selon une ou plusieurs des revendications 8 à 15, **caractérisé par le fait que** la céramique dentaire destinée à recouvrir le titane est appliquée par cuisson sur la couche de titane-silicium (2) en quatre cycles de cuisson

> 1er cycle : Bonder et/ou matière de cuisson de Washbrand
> 2ème cycle : Cuisson de matière de base
> Sème cycle : Cuisson de dentine
> 4ème cycle : Cuisson de brillant

**17.** Procédé de fabrication d'un système composite adhésif titane-céramique selon la revendication 1, **caractérisé par le fait que** pour l'utilisation dans un domaine de hautes températures de 600 à 3600 °C, dans la surface (1) d'un corps en titane pur ou en alliage de titane sont introduits par une implantation ionique avec des faisceaux ioniques (3) entre les atomes (5) du titane ou les atomes (5) de l'alliage de titane des ions silicium (4) qui forment dans la surface (1) du corps dans la couche de pénétration de l'implantation ionique une couche de titane-silicium (2) sur laquelle des matériaux cristallins inorganiques non métalliques sont appliqués par voie thermique et une liaison par adhérence est produite avec ces matériaux.

**18.** Procédé selon la revendication 17, **caractérisé par le fait que** les ions silicium (4) sont introduits dans la couche de titane-silicium (2) sous la forme d'agrégats de silicium.

**19.** Procédé selon la revendication 17, **caractérisé par le fait que** les matériaux cristallins inorganiques non métalliques sont constitués de matériaux vitrocéramiques, de matériaux céramiques non d'oxydes ou de matériaux céramiques d'oxydes.

**20.** Procédé selon la revendication 17, **caractérisé par le fait que** l'implantation ionique d'ions silicium (4) dans la surface (1) du corps en titane pur ou en alliage de titane est effectuée avec une dose ionique de $1 \times 10^8$ à $1 \times 10^{16}$ atomes/cm$^2$ et une énergie ionique de 30 à 400 keV.

**21.** Procédé selon la revendication 20, **caractérisé par le fait que** l'implantation ionique d'ions silicium (4) dans la surface (1) du corps en titane pur ou en alliage de titane est effectuée avec une dose ionique de $9 \times 10^{16}$ atomes/cm$^2$ et une énergie ionique de 150 keV.

**22.** Procédé selon les revendications 17 à 21, **caractérisé par le fait que** le corps en titane pur ou en alliage de titane est réalisé avant l'implantation ionique sous la forme d'une pièce destinée à être utilisée dans un domaine de hautes températures de 600 à 3600 °C, et en dehors de l'application thermique d'un matériau cristallin inorganique non métallique, il n'est pas effectué d'autre traitement du corps après la formation de la couche de titane-silicium (2) dans la surface (1) du corps.

**23.** Procédé selon la revendication 22, **caractérisé par le fait que** la pièce est employée dans des moteurs et des propulseurs de véhicules automobiles ainsi que de l'aéronautique et l'astronautique.

**24.** Système composite adhésif titane-céramique fabriqué par le procédé de la revendication 1, **caractérisé par le fait que** la surface (1) d'un corps en titane pur ou en alliage de titane est réalisée sous la forme d'une couche de titane-silicium (4), les ions silicium (4) étant introduits par une implantation ionique entre les atomes (5) du titane ou les atomes (5) de l'alliage de titane, et sur la couche de titane-silicium (2) étant appliqué par voie thermique un matériau cristallin inorganique non métallique.

**25.** Système composite adhésif titane-céramique selon la revendication 24, **caractérisé par le fait que** les ions silicium (4) pour la formation de la couche de titane-silicium (2) dans la surface (1) du corps en titane pur ou en alliage de titane sont introduits avec une dose ionique de $1 \times 10^8$ à $1 \times 10^{18}$ atomes/cm$^2$ et une énergie ionique de 30 à 400 keV.

**26.** Système composite adhésif titane-céramique selon la revendication 25, **caractérisé par le fait que** les ions silicium (4) dans la surface (1) du corps en titane pur ou en alliage de titane sont introduits avec une dose ionique de $9 \times 10^{16}$ atomes/cm$^2$ et une énergie ionique de 150 keV.

**27.** Système composite adhésif titane-céramique fabriqué par le procédé de la revendication 8, **caractérisé par le fait que** pour l'utilisation pour une prothèse dentaire, la surface (1) d'un corps en titane pur est réalisée sous la forme d'une couche de titane-silicium (2), des ions silicium (4) étant introduits par une implantation ionique entre les atomes de titane (5) du titane pur, et sur la couche de titane-silicium (2) étant appliquée par cuisson une céramique dentaire destinée à recouvrir le titane.

28. Système composite adhésif titane-céramique selon la revendication 27, **caractérisé par le fait que** les ions silicium (4) pour la formation de la couche de titane-silicium (2) dans la surface (1) du corps en titane pur sont introduits avec une dose ionique de 1 x $10^{12}$ à 1 x $10^{16}$ atomes/$cm^2$ d'ions silicium (4) et une énergie ionique de 30 à 400 keV.

29. Système composite adhésif titane-céramique selon la revendication 28, **caractérisé par le fait que** les ions silicium (4) pour la formation de la couche de titane-silicium (2) dans la surface (1) du corps en titane pur sont introduits avec une dose ionique de 3 x $10^{13}$ atomes/$cm^2$ et une énergie ionique de 150 keV.

30. Système composite adhésif titane-céramique selon les revendications 27 à 29, **caractérisé par le fait que** le titane pur a la composition suivante (valeurs en % en masse) :

| | |
|---|---|
| $O_{max}$ | 0,12 |
| $N_{max}$ | 0,05 |
| $C_{max}$ | 0,06 |
| $H_{max}$ | 0,013 |
| Ti | le reste |

31. Système composite adhésif titane-céramique selon une ou plusieurs des revendications 27 à 30, **caractérisé par le fait que** le corps en titane pur est, avant la formation de sa surface (1) sous la forme d'une couche de titane-silicium (2), réalisé entièrement sous la forme d'un corps de base pour une prothèse dentaire, sur lequel, après la formation de la couche de titane-silicium (2), comme seul traitement, la céramique dentaire est appliquée par cuisson.

32. Système composite adhésif titane-céramique selon une ou plusieurs des revendications 27 à 31, **caractérisé par le fait que** la couche de titane-silicium (2) est formée dans toute la surface (1) du corps en titane pur réalisé comme corps de base pour une prothèse dentaire, et la céramique dentaire destinée à recouvrir le titane est appliquée par cuisson sur certaines parties de la surface (1), la céramique dentaire étant appliquée par cuisson au moins sur les parties du corps de base pour une prothèse dentaire qui forment les zones des dents et les zones de contact avec les muqueuses.

33. Système composite adhésif titane-céramique fabriqué par le procédé de la revendication 17, **caractérisé par le fait que** pour l'utilisation dans un domaine de hautes températures de 600 à 3600 °C, la surface (1) d'un corps en titane pur ou en alliage de titane est réalisée sous la forme d'une couche de titane-silicium (2), les ions silicium (4) étant introduits par une implantation ionique entre les atomes (5) du titane ou les atomes (5) de l'alliage de titane, et sur la couche de titane-silicium (2) étant appliqué par voie thermique un matériau cristallin inorganique non métallique.

34. Système composite adhésif titane-céramique selon la revendication 33, **caractérisé par le fait que** les ions silicium (4) sont introduits dans la couche de titane-silicium (2) sous la forme d'agrégats de silicium.

35. Système composite adhésif titane-céramique selon la revendication 33, **caractérisé par le fait que** les matériaux cristallins inorganiques non métalliques sont constitués de matériaux vitrocéramiques, de matériaux céramiques non d'oxydes ou de matériaux céramiques d'oxydes.

36. Système composite adhésif titane-céramique selon la revendication 33, **caractérisé par le fait que** l'implantation ionique d'ions silicium (4) dans la surface (1) du corps en titane pur ou en alliage de titane est effectuée avec une dose ionique de 1 x $10^8$ à 1 x $10^{18}$ atomes/$cm^2$ et une énergie ionique de 30 à 400 keV.

37. Système composite adhésif titane-céramique selon la revendication 36, **caractérisé par le fait que** l'implantation ionique d'ions silicium (4) dans la surface (1) du corps en titane pur ou en alliage de titane est effectuée avec une dose ionique de 9 x $10^{16}$ atomes/$cm^2$ et une énergie ionique de 150keV.

38. Système composite adhésif titane-céramique selon les revendications 33 à 37, **caractérisé par le fait que** le corps en titane pur ou en alliage de titane est, avant l'implantation ionique, réalisé sous la forme d'une pièce destinée à être utilisée dans un domaine de hautes températures de 600 à 3600 °C, et en dehors de l'application thermique d'un matériau cristallin inorganique non métallique, il n'est pas effectué d'autre traitement du corps après la formation de la couche de titane-silicium (2) dans la surface (1) du corps.

39. Système composite adhésif titane-céramique selon la revendication 38, **caractérisé par le fait que** la pièce peut être employée dans des moteurs et des propulseurs de véhicules automobiles ainsi que de l'aéronautique et de l'astronautique.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14